# FASCICULE DE BREVET EUROPEEN

(11) **EP 1 742 964 B1**
(45) Date de publication et mention de la délivrance du brevet: **26.10.2011**
(21) Numéro de dépôt: 05760055.3
(22) Date de dépôt: 21.04.2005
(51) Int. Cl.: C07K 14/47

(54) **PEPTIDE CAPABLE D'ALTERER LA POLYMERISATION DE LA TUBULINE ET SON UTILISATION POUR INHIBER LA PROLIFERATION CELLULAIRE**
PEPTID MIT FÄHIGKEIT ZUR ÄNDERUNG DER TUBULINPOLYMERISATION UND DESSEN VERWENDUNG ZUR HEMMUNG VON ZELLPROLIFERATION
PEPTIDE CAPABLE OF ALTERING TUBULIN POLYMERIZATION AND USE THEREOF FOR INHIBITING CELL PROLIFERATION

(30) Priorité: 04.05.2004 FR 0404742
(43) Date de publication de la demande: 17.01.2007
(73) Titulaire: UNIVERSITE D'ANGERS, 49000 Angers (FR); INSTITUT NATIONAL DE LA SANTE ET DE LA RECHERCHE MEDICALE (INSERM), 75013 Paris (FR); McGill University, Montréal, Québec H3A 1B1 (CA)
(72) Inventeur: BOCQUET, Arnaud, F-49100 Angers (FR); EYER, Joël, F-49320 Blaison-Gohier (FR); PETERSON, Alan, Westmount H3Y 3B4 (CA)
(74) Mandataire: Warcoin, Jacques
(86) Numéro de dépôt international: PCT/FR2005/000990
(87) Numéro de publication internationale: WO 2005/121172

(56) Documents cités:
- WO-A-03/057168
- DATABASE EMBL 1 août 1988 (1988-08-01), XP002308298 extrait de EBI Database accession no. P08551 -& LEWIS S A ET AL: "ANOMALOUS PLACEMENT OF INTRONS IN A MEMBER OF THE INTERMEDIATE FILAMENT MULTIGENE FAMILY AN EVOLUTIONARY CONUNDRUM" MOLECULAR AND CELLULAR BIOLOGY, vol. 6, no. 5, 1986, pages 1529-1534, XP002308295 ISSN: 0270-7306
- DATABASE Geneseq [Online] 20 novembre 2003 (2003-11-20), "Alzheimer's disease-associated protein isoform tryptic peptide #618." XP002308299 extrait de EBI accession no. GSN:ADA24009 Database accession no. ADA24009
- DATABASE UniProt [Online] 1 octobre 1989 (1989-10-01), "Neurofilament triplet M protein (160 kDa neurofilament protein) (Neurofilament medium polypeptide) (NF-M)." XP002308300 extrait de EBI accession no. UNIPROT:NFM_RAT Database accession no. P12839; Q63370 -& NAPOLITANO E W ET AL: "COMPLETE AMINO ACID SEQUENCE AND IN-VITRO EXPRESSION OF RAT NF-M THE MIDDLE MOLECULAR WEIGHT NEUROFILAMENT PROTEIN" JOURNAL OF NEUROSCIENCE, vol. 7, no. 8, 1987, pages 2590-2599, XP009040735 ISSN: 0270-6474
- DATABASE EMBL XP002308301 extrait de EBI Database accession no. Q80TQ3 -& OKAZAKI N ET AL: "Prediction of the coding sequences of mouse homologues of KIAA gene: II. The complete nucleotide sequences of 400 mouse KIAA-homologous cDNAs identified by screening of terminal sequences of cDNA clones randomly sampled from size-fractionated libraries" DNA RESEARCH, UNIVERSAL ACADEMY PRESS, JP, vol. 10, no. 1, 2003, pages 35-48, XP002969478 ISSN: 1340-2838
- HISANAGA S-I ET AL: "DEPHOSPHORYLATION-INDUCED INTERACTIONS OF NEUROFILAMENTS WITH MICROTUBULES" JOURNAL OF BIOLOGICAL CHEMISTRY, vol. 265, no. 35, 1990, pages 21852-21858, XP002308297 ISSN: 0021-9258
- TAMAI Y ET AL: "Sequence of the EndoA gene encoding mouse cytokeratin and its methylation state in the CpG-rich region" GENE 1991 NETHERLANDS, vol. 104, no. 2, 1991, pages 169-176, XP002353420 ISSN: 0378-1119
- KARPOV V ET AL: "Structure of the mouse gene encoding peripherin: A neuronal intermediate filament protein" BIOLOGY OF THE CELL 1992 FRANCE, vol. 76, no. 1, 1992, pages 43-48, XP002353421 ISSN: 0248-4900
- CHIEN CHUNG-LIANG ET AL: "Characterization of the mouse gene encoding the neuronal intermediate filament protein alpha-internexin" GENE (AMSTERDAM), vol. 149, no. 2, 1994, pages 289-292, XP002353422 ISSN: 0378-1119
- BALCAREK J M ET AL: "Structure of the mouse glial fibrillary acidic protein gene: implications for the evolution of the intermediate filament multigene family." NUCLEIC ACIDS RESEARCH. 12 AUG 1985, vol. 13, no. 15, 12 août 1985 (1985-08-12), pages 5527-5543, XP002353423 ISSN: 0305-1048
- CAPETANAKI Y ET AL: "Mouse vimentin: Structural relationship to fos, jun, CREB and tpr" ONCOGENE 1990 UNITED KINGDOM, vol. 5, no. 5, 1990, pages 645-655, XP009056912 ISSN: 0950-9232
- MERICSKAY M ET AL: "An overlapping CArG/octamer element is required for regulation of desmin gene transcription in arterial smooth muscle cells" DEVELOPMENTAL BIOLOGY 15 OCT 2000 UNITED STATES, vol. 226, no. 2, 15 octobre 2000 (2000-10-15), pages 192-208, XP002286355 ISSN: 0012-1606
- STRAUSBERG R L ET AL: "Generation and initial analysis of more than 15,000 full-length human and mouse cDNA sequences" PROCEEDINGS OF THE NATIONAL ACADEMY OF SCIENCES OF THE UNITED STATES OF AMERICA 24 DEC 2002 UNITED STATES, vol. 99, no. 26, 24 décembre 2002 (2002-12-24), pages 16899-16903, XP002353426 ISSN: 0027-8424

## Description

La présente invention a pour objet un peptide issu de filaments intermédiaires et un fragment de filament intermédiaire capables d'altérer la polymérisation de la tubuline et utilisés pour inhiber la prolifération cellulaire, et plus particulièrement pour l'obtention de médicaments destinés à prévenir ou traiter les maladies impliquant une prolifération cellulaire, comme par exemple les cancers.

La division cellulaire, ou mitose, est le processus qui permet aux cellules de se multiplier afin de construire ou régénérer les tissus et de remplacer les cellules mortes. Chez les cellules cancéreuses la régulation de ce processus est défaillante, c'est pourquoi ces cellules se divisent de manière anarchique et créent ainsi des tumeurs. L'une des voies thérapeutiques efficace pour lutter contre les cancers consiste à bloquer la division des cellules cancéreuses.

Les molécules antimitotiques actuelles (Taxol ou Colchicine) agissent sur toutes les cellules sans distinction et provoquent par conséquent de nombreux effets secondaires.

Il est donc essentiel de développer des molécules anti-mitotiques, c'est-à-dire des molécules bloquant la division des cellules cancéreuses, comportant beaucoup moins d'effet secondaire.

La tubuline est une molécule fondamentale du processus de la division cellulaire. En effet, elle se polymérise pour former les microtubules qui sont indispensables au transport intracellulaire lors de la mitose.

Les microtubules sont un des éléments les plus importants du cytosquelette axonal où ils jouent un rôle essentiel dans le transport axonal fonctionnant comme des rails le long desquels les organelles bougent dans les deux directions antérogrades et rétrogrades grâce à des protéines motrices spécifiques. Les microtubules sont en équilibre dynamique avec les dimères de tubuline et leur assemblage est régulé dans une large mesure grâce à l'association des protéines additionnelles qui permettent d'augmenter ou de réduire la stabilité des microtubules comme par exemple les protéines MAPs (revu par Mandelkow et Mandelkow, 1995), les protéines STOP (Guillaud et al., 1998) et les protéines OP18 (Cassimeris, 2002). Les axones dépendent des microtubules pour le transport axoplasmique et des neurofilaments pour leur croissance radiale.

Les neurofilaments (NFs) sont la classe majoritaire des filaments intermédiaires exprimée par les neurones matures (Hoffman et al., 1987). Ils sont composés de trois sous-unités indépendamment encodées, les neurofilaments légers (NFL), les neurofilaments médium (NFM) et les neurofilaments lourds (NFH) de 68, 150 et 200 kDa respectivement. L'axe de chaque neurofilament est formé par des sous unités NFL. Les sous-unités NFM et NFH se fixent sur cet axe grâce à des interactions de types hélice-hélice situées dans leurs domaines centraux. Les domaines carboxy-terminaux de NFM et NFH forment des projections latérales particulièrement longues qui permettent les interactions interfilaments et l'association de neurofilaments avec d'autres organelles (Hirokawa, 1982). L'état de phosphorylation des motifs KSP répétés dans ces projections latérales (Julien et Mushynski, 1982 ; De Waegh et al, 1992 ; Lee et al., 1988) est connu pour moduler l'affinité interfilaments (Eyer et Leterrier, 1988), l'espacement interfilaments (Carden et al., 1987), l'interaction avec la tubuline (Hisanaga et Hirokawa, 1990), la vitesse de transport des neurofilaments (De Waegh et al., 1992) et la croissance radiale des axones (Ohara et al., 1993 ; Eyer et Peterson, 1994 ; Zhu et al., 1997).

Il est connu que les filaments intermédiaires ont la particularité d'être différents d'un tissu à l'autre. Ainsi, les neurofilaments sont présents uniquement dans les cellules neuronales, les gliofilaments dans les cellules gliales, les filaments de desmine dans les cellules musculaires, et les filaments de kératines dans les cellules de l'épiderme.

De manière surprenante et inattendue, les inventeurs ont montré que des fragments de filaments intermédiaires peuvent lier la tubuline et altérer la polymérisation des microtubules. De plus, les inventeurs ont montré que l'utilisation d'un peptide liant la tubuline et inhibant ou activant la polymérisation des microtubules permet de bloquer la division cellulaire.

En outre, lesdits peptides sont issus de la séquence de filaments intermédiaires, lesquels sont tissus-spécifiques. Ces peptides ne semblent donc pas provoquer les effets secondaires de molécules anti-mitotiques actuelles.

L'invention a donc pour objet

un peptide isolé caractérisé en ce qu'il consiste en un peptide présentant au moins 80 % de préférence 85 %, 90 %, 95 % 98 % et 100% d'identité après alignement optimal avec une séquence choisie parmi SEQ ID N°5 (FGYDPYFSTSYKRRYVETPRVHIS), SEQ ID N°6 (YSSYSAPVSSSLSVRRSYSSSSGS), SEQ ID N°7 (AYRRVPTETRSSFSRVSGSPSSGFRSQSWSRGSPSTVSS), SEQ ID N°8 (RSAAGSSSGFHSWARTSVSSVSASPSRFRGAASSTDSLD), SEQ ID N°17 (MSQAYSSSQRVSSYRRTFGGAPGFSLG), SEQ ID N° 18 (SSPVFPRAGFGTKGSSSSMTSRVYQVSRTSGGAGGLGSLRSSRLGTTRAP SYGA), SEQ ID N° 19 (GSEVHTKKTVMIKTIETRDGE), SEQ ID N° 20 (MSTRSVSSSSYRRMFGGS), SEQ ID N°21 (GGAYVTRSSAVRLRSSVPGVRLLQ), SEQ ID N°22 (SLPLVDTHSKRTLLIKTVETR), SEQ ID N°23 (MSIRVTQKSYKMSTSGPRAFSSRSFTSGPGARISSSSFSRVGSSSSSFRGSM GT), SEQ ID N°24 (QIKSLNNKFASFIDKVRFLEQ), SEQ ID N°25 (SAGGSNTFSRTTKAVVVKKIETRDGKLVSE), SEQ ID N°26 (MERRRITSARRSYASETVVRGLGP), SEQ ID N°27 (VRGLGPSRQLGTMPRFSLSRMTPPLPARVDFSLAGA), SEQ ID N°28 (KSVSEGHLKRNIVVKTVEMRD) ou l'un de ses fragments biologiques artificiel d'au moins 5 aminoacides capable d'altérer la polymérisation de la tubuline.

Par « altérer la polymérisation de la tubuline », on entend soit inhiber soit activer la polymérisation de la tubuline.

Dans la présente invention, on entendra désigner par le terme « peptide », également les polypeptides.

Avantageusement, ledit peptide présente au moins 90 % d'identité après alignement optimal avec une séquence choisie parmi SEQ ID N°5, SEQ ID N°6, SEQ ID N°7, SEQ ID N°8, SEQ ID N°17, SEQ ID N°18, SEQ ID N°19, SEQ ID N°20, SEQ ID N°21, SEQ ID N°22, SEQ ID N°23, SEQ ID N°24, SEQ ID N°25, SEQ ID N°26, SEQ ID N°27 ou SEQ ID N°28. De manière encore plus avantageuse, ledit peptide présente au moins 100% d'identité après alignement optimal avec une séquence choisie parmi SEQ ID N°5, SEQ ID N°6, SEQ ID N°7, SEQ ID N°8, SEQ ID N°17, SEQ ID N°18, SEQ ID N°19, SEQ ID N°20, SEQ ID N°21, SEQ ID N°22, SEQ ID N°23, SEQ ID N°24, SEQ ID N°25, SEQ ID N°26, SEQ ID N°27 ou SEQ ID N°28 et des combinaisons de ceci.

Par « pourcentage d'identité » entre deux séquences d'acide nucléique ou d'acide aminé au sens de la présente invention, on entend désigner un pourcentage de nucléotides ou de résidus d'acides aminés identiques entre les deux séquences à comparer, obtenu après le meilleur alignement (alignement optimal), ce pourcentage étant purement statistique et les différences entre les deux séquences étant réparties au hasard et sur toute leur longueur. Les comparaisons de séquences entre deux séquences d'acide nucléique ou d'acide aminé sont traditionnellement réalisées en comparant ces séquences après les avoir alignées de manière optimale, ladite comparaison pouvant être réalisée par segment ou par «fenêtre de comparaison». L'alignement optimal des séquences pour la comparaison peut être réalisé, outre manuellement, au moyen de l'algorithme d'homologie locale de Smith et Waterman (1981) [Ad. App. Math. 2:482], au moyen de l'algorithme d'homologie locale de Neddleman et Wunsch (1970) [J. Mol. Biol. 48:443], au moyen de la méthode de recherche de similarité de Pearson et Lipman (1988) [Proc. Natl. Acad. Sci. USA 85:2444], au moyen de logiciels informatiques utilisant ces algorithmes (GAP, BESTFIT, FASTA et TFASTA dans le Wisconsin Genetics Software Package, Genetics Computer Group, 575 Science Dr., Madison, WI, ou encore par les logiciels de comparaison BLAST N, ou BLAST P, ClustalW).

Le pourcentage d'identité entre deux séquences d'acide nucléique ou d'acide aminé est déterminé en comparant ces deux séquences alignées de manière optimale dans laquelle la séquence d'acide nucléique ou d'acide aminé à comparer peut comprendre des additions ou des délétions par rapport à la séquence de référence pour un alignement optimal entre ces deux séquences. Le pourcentage d'identité est calculé en déterminant le nombre de positions identiques pour lesquelles le nucléotide ou le résidu d'acide aminé est identique entre les deux séquences, en divisant ce nombre de positions identiques par le nombre total de positions dans la fenêtre de comparaison et en multipliant le résultat obtenu par 100 pour obtenir le pourcentage d'identité entre ces deux séquences.

Par exemple, on pourra utiliser le programme BLAST, « BLAST 2 séquences » (Tatusova et al., "Blast 2 séquences - a new tool for comparing protein and nucleotide séquences", FEMS Microbiol Lett. 174:247-250) disponible sur le site http://www.ncbi.nlm.nih.gov/gorf/bl2.html, les paramètres utilisés étant ceux donnés par défaut (en particulier pour les paramètres « open gap penaltie » : 5, et « extension gap penaltie » : 2 ; la matrice choisie étant par exemple la matrice « BLOSUM 62 » proposée par le programme), le pourcentage d'identité entre les deux séquences à comparer étant calculé directement par le programme.

Par séquence d'acide aminé présentant au moins 80 %, de préférence 85 %, 90 %, 95 % et 98 % d'identité avec une séquences d'acide aminé de référence, on préfère celles présentant par rapport à la séquence de référence, certaines modifications, en particulier une délétion, addition ou substitution d'au moins un acide aminé, une troncation ou un allongement. Dans le cas d'une substitution, d'un ou plusieurs acide(s) aminé(s) consécutif(s) ou non consécutif(s), on préfère les substitutions dans lesquelles les acides aminés substitués sont remplacés par des acides aminés « équivalents ». L'expression « acides aminés équivalents » vise ici à désigner tout acide aminé susceptible d'être substitué à l'un des acides aminés de la structure de base sans cependant modifier essentiellement les activités biologiques des anticorps correspondants et telles qu'elles seront définies par la suite, notamment dans les exemples.

Ces acides aminés équivalents peuvent être déterminés soit en s'appuyant sur leur homologie de structure avec les acides aminés auxquels ils se substituent, soit sur des résultats d'essais comparatifs d'activité biologique entre les différents peptides et/ou entre les différents fragments de filaments intermédiaires.

A titre d'exemple, on mentionne les possibilités de substitution susceptibles d'être effectuées sans qu'il résulte en une modification approfondie de l'activité biologique du peptide modifié correspondant. On peut remplacer ainsi la leucine par la valine ou l'isoleucine, l'acide aspartique par l'acide glutamine, la glutamine par l'asparagine, l'arginine par la lysine, etc., les substitutions inverses étant naturellement envisageables dans les mêmes conditions. Enfin, les modifications des acides aminés (par exemple phosphorylation, biotinylation, acétylation...) qui sont susceptibles de rendre l'activité biologique de ces peptides plus efficace sont aussi envisagées.

L'invention a également pour objet un peptide de séquence choisie parmi SEQ ID N°5, SEQ ID N°6, SEQ ID N°7, SEQ ID N°8, SEQ ID N°17, SEQ ID N°18, SEQ ID N°19, SEQ ID N°20, SEQ ID N°21, SEQ ID N°22, SEQ ID N°23, SEQ ID N°24, SEQ ID N°25, SEQ ID N°26, SEQ ID N°27 ou SEQ ID N°28.

La présente invention a également pour objet un peptide de séquence SEQ ID n°5, nommé Neurofilament Light subunit-Tubulin Binding Site 1 (NFL-TBS1). Par NFL-TBS1, on entend désigner le polypeptide responsable de la liaison à la tubuline issue de la sous unité de neurofilament de faible poids moléculaire capable de lier la tubuline.

Par faible poids moléculaire, on entend entre 1 et 70 kDa, préférentiellement 34 kDa.

La présente invention a également pour objet un peptide de séquence SEQ ID n°6, nommé Neurofilament Light subunit-Tubulin Binding Site 2 (NFL-TBS2). Par NFL-TBS2, on entend désigner le polypeptide responsable de la liaison à la tubuline issue de la sous unité de neurofilament de faible poids moléculaire capable de lier la tubuline.

Par faible poids moléculaire, on entend entre 1 et 70 kDa, préférentiellement 34 kDa.

La présente invention a également pour objet un peptide de séquence SEQ ID n°7, nommé Neurofilament Medium subunit-Tubulin Binding Site (NFM-TBS). Par NFM-TBS, on entend désigner le polypeptide responsable de la liaison à la tubuline issue de la sous unité de neurofilament de poids moléculaire moyen capable de lier la tubuline.

Par poids moléculaire moyen on entend entre 1 et 170 kDa, préférentiellement 150 kDa.

La présente invention a également pour objet un peptide de séquence SEQ ID n°8, nommé Neurofilament high subunit-Tubulin Binding Site (NFH-TBS). Par NFH-TBS, on entend désigner le polypeptide responsable de la liaison à la tubuline issue de la sous unité de neurofilament de poids moléculaire élevé capable de lier la tubuline.

Par poids moléculaire important on entend un poids moléculaire supérieur à 170kDa, préférentiellement 200 kDa.

La présente invention a également pour objet un peptide de séquence SEQ ID N°17, nommé Desmine-Tubulin Binding Site (Des-TBS1). Par Des-TBS1, on entend désigner le polypeptide responsable de la liaison à la tubuline issue de la Desmine, capable de lier la tubuline.

La présente invention a également pour objet un peptide de séquence SEQ ID N°18, nommé Desmine-Tubulin Binding Site (Des-TBS2). Par Des-TBS2, on entend désigner le polypeptide responsable de la liaison à la tubuline issue de la Desmine, capable de lier la tubuline.

La présente invention a également pour objet un peptide de séquence SEQ ID N°19, nommé Desmine-Tubulin Binding Site (Des-TBS3). Par Des-TBS3, on entend désigner le polypeptide responsable de la liaison à la tubuline issue de la Desmine, capable de lier la tubuline.

La présente invention a également pour objet un peptide de séquence SEQ ID N°20, nommé Vimentine-Tubulin Binding Site (Vim-TBS1). Par Vim-TBS1, on entend désigner le polypeptide responsable de la liaison à la tubuline issue de la Vimentine capable de lier la tubuline.

La présente invention a également pour objet un peptide de séquence SEQ ID N°21, nommé Vimentine-Tubulin Binding Site (Vim-TBS2). Par Vim-TBS2, on entend désigner le polypeptide responsable de la liaison à la tubuline issue de la Vimentine capable de lier la tubuline.

La présente invention a également pour objet un peptide de séquence SEQ ID N°22, nommé Vimentine-Tubulin Binding Site (Vim-TBS3). Par Vim-TBS3, on entend désigner le polypeptide responsable de la liaison à la tubuline issue de la Vimentine capable de lier la tubuline.

La présente invention a également pour objet un peptide de séquence SEQ ID N°23, nommé Cytokeratine-Tubulin Binding Site (Ker-TBS1). Par Ker-TBS1, on entend désigner le polypeptide responsable de la liaison à la tubuline issue de la Cytokeratine, capable de lier la tubuline.

La présente invention a également pour objet un peptide de séquence SEQ ID N°24, nommé Cytokeratine-Tubulin Binding Site (Ker-TBS2). Par Ker-TBS2, on entend désigner le polypeptide responsable de la liaison à la tubuline issue de la Cytokeratine, capable de lier la tubuline.

La présente invention a également pour objet un peptide de séquence SEQ ID N°25, nommé Cytokeratine-Tubulin Binding Site (Ker-TBS3). Par Ker-TBS3, on entend désigner le polypeptide responsable de la liaison à la tubuline issue de la Cytokeratine, capable de lier la tubuline.

La présente invention a également pour objet un peptide de séquence SEQ ID N°26, nommé Glial Fibrillary Acidic Protein-Tubulin Binding Site (GFAP-TBS1). Par GFAP-TBS1, on entend désigner le polypeptide responsable de la liaison à la tubuline issue de GFAP, capable de lier la tubuline.

La présente invention a également pour objet un peptide de séquence SEQ ID N°27, nommé Glial Fibrillary Acidic Protein-Tubulin Binding Site (GFAP-TBS2). Par GFAP-TBS2, on entend désigner le polypeptide responsable de la liaison à la tubuline issue de GFAP, capable de lier la tubuline.

La présente invention a également pour objet un peptide de séquence SEQ ID N°28, nommé Glial Fibrillary Acidic Protein-Tubulin Binding Site (GFAP-TBS3). Par GFAP-TBS3, on entend désigner le polypeptide responsable de la liaison à la tubuline issue de GFAP, capable de lier la tubuline.

Selon la présente invention, lesdits peptides peuvent notamment être obtenus par synthèse chimique peptidique classique ou par voie recombinante, connues de l'homme du métier.

Les méthodes de préparation des peptides recombinants sont aujourd'hui bien connues de l'homme du métier et ne seront pas développées dans la présente description. Parmi les cellules utilisables pour la production de ces protéines recombinantes, on peut notamment citer les cellules bactériennes, et plus particulièrement E. coli.

La mesure de l'altération de la polymérisation de la tubuline est faite en mesurant la turbidité (densité optique à 350 nm) d'une suspension de tubuline. Une diminution d'au moins 1% de la turbidité est signe d'une inhibition de la polymérisation de la tubuline.

La présente invention a également pour objet un acide nucléique isolé codant pour un des peptides selon la présente invention.

Par acide nucléique, on entend désigner selon la présente invention de l'ADN ou de l'ARN.

L'invention a donc pour objet un acide nucléique présentant au moins 80 %, de préférence 85 %, 90 %, 95, % 98 % et 100% d'identité après alignement optimal avec une séquence choisie parmi SEQ ID N°1, SEQ ID N°2, SEQ ID N°3, SEQ ID N°4, SEQ ID N°29, SEQ ID N°30, SEQ ID N°31, SEQ ID N°32, SEQ ID N°33, SEQ ID N°34, SEQ ID N°35, SEQ ID N°36, SEQ ID N°37, SEQ ID N°38, SEQ ID N°39 ou SEQ ID N°40.

Plus particulièrement, la présente invention a pour objet un acide nucléique présentant au moins 80 %, de préférence 85 %, 90 %, 95, % 98 % et 100% d'identité après alignement optimal avec la séquence SEQ ID N°1, codant pour un peptide de séquence d'acides aminés SEQ ID n°5, nommé NFL-TBS1.

Plus particulièrement, la présente invention a pour objet un acide nucléique présentant au moins 80 %, de préférence 85 %, 90 %, 95, % 98 % et 100% d'identité après alignement optimal avec la séquence SEQ ID N°2, codant pour un peptide de séquence d'acides aminés SEQ ID n°6, nommé NFL-TBS2.

Plus particulièrement, la présente invention a pour objet un acide nucléique présentant au moins 80 %, de préférence 85 %, 90 %, 95 %, 98 % et 100% d'identité après alignement optimal avec la séquence SEQ ID N°3, codant pour un peptide de séquence d'acides aminés SEQ ID n°7, nommé NFM-TBS.

Plus particulièrement, la présente invention a pour objet un acide nucléique présentant au moins 80 %, de préférence 85 %, 90 %, 95 %, 98 % et 100% d'identité après alignement optimal avec la séquence SEQ ID N°4, codant pour un peptide de séquence d'acides aminés SEQ ID n°8, nommé NFH-TBS.

Plus particulièrement, la présente invention a pour objet un acide nucléique présentant au moins 80 %, de préférence 85 %, 90 %, 95 %, 98 % et 100% d'identité après alignement optimal avec la séquence SEQ ID N°29, codant pour un peptide de séquence d'acides aminés SEQ ID n°17, nommé Des-TBS1.

Plus particulièrement, la présente invention a pour objet un acide nucléique présentant au moins 80 %, de préférence 85 %, 90 %, 95 %, 98 % et 100% d'identité après alignement optimal avec la séquence SEQ ID N°30, codant pour un peptide de séquence d'acides aminés SEQ ID n°18, nommé Des- TBS2.

Plus particulièrement, la présente invention a pour objet un acide nucléique présentant au moins 80 %, de préférence 85 %, 90 %, 95 %, 98 % et 100% d'identité après alignement optimal avec la séquence SEQ ID N°31, codant pour un peptide de séquence d'acides aminés SEQ ID n°19, nommé Des- TBS3.

Plus particulièrement, la présente invention a pour objet un acide nucléique présentant au moins 80 %, de préférence 85 %, 90 %, 95 %, 98 % et 100% d'identité après alignement optimal avec la séquence SEQ ID N°32, codant pour un peptide de séquence d'acides aminés SEQ ID n°20, nommé Vim- TBS1.

Plus particulièrement, la présente invention a pour objet un acide nucléique présentant au moins 80 %, de préférence 85 %, 90 %, 95 %, 98 % et 100% d'identité après alignement optimal avec la séquence SEQ ID N°33, codant pour un peptide de séquence d'acides aminés SEQ ID n°21, nommé Vim- TBS2.

Plus particulièrement, la présente invention a pour objet un acide nucléique présentant au moins 80 %, de préférence 85 %, 90 %, 95 %, 98 % et 100% d'identité après alignement optimal avec la séquence SEQ ID N°34, codant pour un peptide de séquence d'acides aminés SEQ ID n°22, nommé Vim- TBS3.

Plus particulièrement, la présente invention a pour objet un acide nucléique présentant au moins 80 %, de préférence 85 %, 90 %, 95 %, 98 % et 100% d'identité après alignement optimal avec la séquence SEQ ID N°35, codant pour un peptide de séquence d'acides aminés SEQ ID n°23, nommé Ker- TBS1.

Plus particulièrement, la présente invention a pour objet un acide nucléique présentant au moins 80 %, de préférence 85 %, 90 %, 95 %, 98 % et 100% d'identité après alignement optimal avec la séquence SEQ ID N°36, codant pour un peptide de séquence d'acides aminés SEQ ID n°24, nommé Ker- TBS2.

Plus particulièrement, la présente invention a pour objet un acide nucléique présentant au moins 80 %, de préférence 85 %, 90 %, 95 %, 98 % et 100% d'identité après alignement optimal avec la séquence SEQ ID N°37, codant pour un peptide de séquence d'acides aminés SEQ ID n°25, nommé Ker- TBS3.

Plus particulièrement, la présente invention a pour objet un acide nucléique présentant au moins 80 %, de préférence 85 %, 90 %, 95 %, 98 % et 100% d'identité après alignement optimal avec la séquence SEQ ID N°38, codant pour un peptide de séquence d'acides aminés SEQ ID n°26, nommé GFAP- TBS1.

Plus particulièrement, la présente invention a pour objet un acide nucléique présentant au moins 80 %, de préférence 85 %, 90 %, 95 %, 98 % et 100% d'identité après alignement optimal avec la séquence SEQ ID N°39, codant pour un peptide de séquence d'acides aminés SEQ ID n°27, nommé GFAP- TBS2. Plus particulièrement, la présente invention a pour objet un acide nucléique présentant au moins 80 %, de préférence 85 %, 90 %, 95 %, 98 % et 100% d'identité après alignement optimal avec la séquence SEQ ID N°40, codant pour un peptide de séquence d'acides aminés SEQ ID n°28, nommé GFAP- TBS3.

La présente invention a également pour objet un vecteur qui comprend un acide nucléique selon la présente invention. Ledit acide nucléique est avantageusement choisi parmi le groupe comprenant SEQ ID N°1, SEQ ID N°2, SEQ ID N°3, SEQ ID N°4, SEQ ID N°29, SEQ ID N°30, SEQ ID N°31, SEQ ID N°32, SEQ ID N°33, SEQ ID N°34, SEQ ID N°35, SEQ ID N°36, SEQ ID N°37, SEQ ID N°38, SEQ ID N°39 et SEQ ID N°40.

Par vecteur, on entend désigner selon la présente invention un plasmide, un cosmide, un phage, un BAC (Bacterial Artificial Chromosome), un YAC (Yeast Artificial Chromosome) ou tout autre fragment d'ADN capable de se repliquer dans une cellule.

La présente invention a également pour objet une cellule hôte qui est transformée par un acide nucléique selon la présente invention ou un vecteur selon la présente invention. Ledit acide nucléique est avantageusement choisi parmi le groupe comprenant SEQ ID N°1, SEQ ID N°2, SEQ ID N°3, SEQ ID N°4, SEQ ID N°29, SEQ ID N°30, SEQ ID N°31, SEQ ID N°32, SEQ ID N°33, SEQ ID N°34, SEQ ID N°35, SEQ ID N°36, SEQ ID N°37, SEQ ID N°38, SEQ ID N°39 et SEQ ID N°40.

Par cellule hôte, on entend désigner selon la présente invention aussi bien des cellules eucaryotes que procaryotes. Parmi les cellules eucaryotes, qui conviennent, on peut citer les cellules animales, végétales, bactériennes les levures, les champignons ou les cellules embryonnaires souches (souris, rat, humaine, et de toutes les espèces en général). En particulier, s'agissant de levures, on peut citer les levures du genre Saccharomyces, Kluyveromyces, Pichia, Schwanniomyces ou Hansenula. S'agissant de cellules animales, on peut citer les cellules COS, CHO, C127, les oeufs de Xénope, etc. Parmi les champignons, on peut citer plus particulièrement Micromonospora, Aspergillus ssp. ou Trichoderma ssp. Comme cellules procaryotes, on préfère utiliser les bactéries suivantes Actinomycètes, et notatnment Streptomyces, E. coli, ou

### Bacillus.

Les cellules hôtes selon la présente invention peuvent être transformées par toute méthode permettant d'introduire une séquence nucléique étrangère dans une cellule. Il peut s'agir notamment de transformation, électroporation, conjugaison, fusion de protoplastes, ou toute autre technique connue de l'homme de l'art.

La présente invention a également pour objet un procédé de production de peptide recombinant selon la présente invention, caractérisé en ce qu'il comprend les étapes suivantes :
a) culture d'une cellule selon la présente invention dans un milieu de culture et des conditions de culture appropriées,
b) récupération dudit peptide à partir des cellules ou du milieu de culture obtenu à l'étape a).

La présente invention a également pour objet une composition pharmaceutique comprenant un peptide selon la présente invention et/ou obtenu par un procédé selon la présente invention, avec un véhicule pharmaceutiquement acceptable: Par véhicule pharmaceutiquement acceptable, on entend désigner selon la présente invention, les supports et véhicules administrables à l'être humain ou à un animal.

La présente invention a également pour objet un peptide selon la présente invention et/ou obtenu par un procédé selon la présente invention pour une utilisation pour inhiber la prolifération cellulaire et/ou la mobilité cellulaire.

Par prolifération cellulaire, on entend désigner selon la présente invention une suite de divisions rapides de cellules ou de microorganismes. Par microorganismes on entend également les endoparasites.

Par cellules on entend notamment toutes les cellules qui ont un cytosquelette de microtubule, par exemple les cellules d'un tissu donné comme le poumon, le foie, le sein, ou les vaisseaux sanguins mais aussi les cellules libres comme les spermatozoïdes, les ovules, ou les plaquettes sanguines.

La division de cellules aboutit à des populations cellulaires homogènes, qui peuvent être organisées en tissus ou non organisées en tissus. Elle s'accompagne parfois de l'apparition d'une certaine anarchie de structure, pouvant aller jusqu'à la perte de la forme et de propriétés caractéristiques. Des proliférations de cellules s'observent notamment au cours de processus de nature inflammatoire ou tumorale.

Ce phénomène, normal au cours du développement et de la croissance pour la plupart des tissus et d'une façon permanente pour certaines lignées cellulaires (éléments figurés du sang, lignée spermatique, etc.), devient anormal dans certaines conditions; il conduit à la formation de tissus néoformés ou néoplasiques.

Un processus essentiel à la croissance tumorale et impliquant une prolifération cellulaire est l'angiogénèse. Un peptide selon l'invention peut donc notamment être utilisé pour inhiber l'angiogénèse au cours de la croissance tumorale. Egalement, le peptide selon la présente invention peut être utilisé pour inhiber la multiplication bactérienne ou la prolifération de certaines plantes comme les algues ou encore les lichens ou des champignons, qui ont un cytosquelette de microtubule. Plus particulièrement, le peptide selon la présente invention peut être utilisé pour inhiber le développement de parasites et plus particulièrement d'endoparasites dans l'alimentation.

La tubuline, une fois polymérisée en microtubules joue un rôle essentiel dans le transport axonal fonctionnant comme des rails le long desquels les organelles bougent dans les deux directions antérogrades et rétrogrades grâce à des protéines motrices spécifiques. Le peptide selon la présente invention est capable de traverser la membrane cellulaire. De plus, ledit peptide bloquant la polymérisation de la tubuline en microtubule, et ayant la capacité de se fixer à la tubuline, il permet de bloquer le transport intracellulaire et la fixation de protéines motrices.

Ainsi, la présente invention a également pour objet un peptide selon la présente invention et/ou obtenu par procédé selon la présente invention pour une utilisation pour bloquer le transport intracellulaire et/ou la mobilité cellulaire.

En outre, ledit peptide bloque la polymérisation de la tubuline en microtubule en se liant à la tubuline. De ce fait, il est possible grâce à cette liaison de marquer la tubuline et les microtubules par ledit peptide.

Ainsi, la présente invention a également pour objet un peptide selon la présente invention et/ou obtenu par procédé selon la présente invention pour une utilisation comme marqueur de la tubuline et des microtubules.

La présente invention a également pour objet l'utilisation d'un peptide selon la présente invention et/ou obtenu par un procédé selon la présente invention, pour l'obtention de médicaments.

Selon la présente invention, le médicament est destiné à prévenir ou traiter des maladies impliquant une prolifération cellulaire et/ou une motilité cellulaire.

Par maladies impliquant une prolifération cellulaire et/ou une motilité cellulaire, on peut citer par exemple, selon la présente invention les cancers, les infections bactériennes ou les maladies impliquant une prolifération de certaines plantes comme les algues ou encore les lichens ou le psoriasis.

Plus particulièrement, selon la présente invention, le médicament est destiné à prévenir ou traiter des cancers.

Par cancer, on entend désigner selon la présente invention une ou plusieurs tumeur(s) composée(s) de cellules atypiques, caractérisées par un pouvoir d'accroissement autonome, une délimitation imprécise, une capacité d'envahissement des tissus et vaisseaux voisins et une tendance à disséminer par la production de métastases.

Il existe deux grandes catégories de tumeurs malignes : les carcinomes, d'origine épithéliale, et les sarcomes, d'origine conjonctive. Il existe également des tumeurs embryonnaires. Le terme cancer est un terme générique désignant toutes les formations néoplasiques malignes, quelle qu'en soit la nature histologique.

De façon encore plus particulière, selon la présente invention, le médicament est destiné à prévenir ou traiter le cancer du système nerveux, le cancer du foie, le cancer de la prostate ou le cancer de la peau.

De manière préférée, selon la présente invention, le cancer est un cancer du système nerveux.

De plus, le peptide selon la présente invention a un effet au niveau des sites d'interaction entre la tubuline et les filaments intermédiaires, sites susceptibles d'être mutés dans des maladies génétiques. En effet, dans le cas où l'interaction entre la tubuline et un filament intermédiaire est nécessaire à l'activation d'une enzyme (par exemple une kinase ou une phosphatase lors de la division normale), une mutation au niveau du site d'interaction empêche l'activation de ladite enzyme. L'ajout d'un peptide normal permet alors de rétablir une interaction entre la tubuline et le peptide et donc l'activation de l'enzyme.

La présente invention a également pour objet l'utilisation d'un peptide selon la présente invention et/ou obtenu par un procédé selon la présente invention pour la mise en évidence et/ou pour tester in vitro des produits susceptibles de bloquer l'interaction entre la tubuline et ledit peptide.

La présente invention a également pour objet l'utilisation d'un fragment de filament intermédiaire capable d'altérer la polymérisation de la tubuline choisi parmi la cytokératine endoA présentant au moins 80%, de préférence 85%, 90%, 98% et 100% d'identité après alignement optimal avec la séquence SEQ ID N°9, la kératine 8 présentant au moins 80%, de préférence 85%, 90%, 98% et 100% d'identité après alignement optimal avec la séquence SEQ ID N°10, la périphérine présentant au moins 80%, de préférence 85%, 90%, 98% et 100% d'identité après alignement optimal avec la séquence SEQ ID N°11, l'internexine présentant au moins 80%, de préférence 85%, 90%, 98% et 100% d'identité après alignement optimal avec la séquence SEQ ID N°12, la GFAP présentant au moins 80%, de préférence 85%, 90%, 98% et 100% d'identité après alignement optimal avec la séquence SEQ ID N°13 ou la vimentine présentant au moins 80%, de préférence 85%, 90%, 98% et 100% d'identité après alignement optimal avec la séquence SEQ ID N°14 pour la fabrication d'un médicament destiné à prévenir ou à traiter des maladies impliquant la prolifération cellulaire et/ou la mobilité cellulaire.

La présente invention a également pour objet l'utilisation d'un fragment de filament intermédiaire capable d'altérer la polymérisation de la tubuline choisi parmi la cytokératine endoA présentant au moins 80%, de préférence 85%, 90%, 98% et 100% d'identité après alignement optimal avec la séquence SEQ ID N°9, la kératine 8 présentant au moins 80%, de préférence 85%, 90%, 98% et 100% d'identité après alignement optimal avec la séquence SEQ ID N°10, la périphérine présentant au moins 80%, de préférence 85%, 90%, 98% et 100% d'identité après alignement optimal avec la séquence SEQ ID N°11, l'internexine présentant au moins 80%, de préférence 85%, 90%, 98% et 100% d'identité après alignement optimal avec la séquence SEQ ID N°12, la GFAP présentant au moins 80%, de préférence 85%, 90%, 98% et 100% d'identité après alignement optimal avec la séquence SEQ ID N°13 ou la vimentine présentant au moins 80%, de préférence 85%, 90%, 98% et 100% d'identité après alignement optimal avec la séquence SEQ ID N°14 pour la fabrication d'un médicament destiné à prévenir où à traiter des maladies impliquant le transport intracellulaire et la fixation de protéines motrices.

La présente invention a également pour objet l'utilisation d'un fragment de filament intermédiaire capable d'altérer la polymérisation de la tubuline choisi parmi la cytokératine endoA présentant au moins 80%, de préférence 85%, 90%, 98% et 100% d'identité après alignement optimal avec la séquence SEQ ID N°9, la kératine 8 présentant au moins 80%, de préférence 85%, 90%, 98% et 100% d'identité après alignement optimal avec la séquence SEQ ID N°10, la périphérine présentant au moins 80%, de préférence 85%, 90%, 98% et 100% d'identité après alignement optimal avec la séquence SEQ ID N°11, l'internexine présentant au moins 80%, de préférence 85%, 90%, 98% et 100% d'identité après alignement optimal avec la séquence SEQ ID N°12, la GFAP présentant au moins 80%, de préférence 85%, 90%, 98% et 100% d'identité après alignement optimal avec la séquence SEQ ID N°13 ou la vimentine présentant au moins 80%, de préférence 85%, 90%, 98% et 100% d'identité après alignement optimal avec la séquence SEQ ID N°14 comme marqueur de la tubuline.

La présente invention a également pour objet l'utilisation d'un fragment de filament intermédiaire capable d'altérer la polymérisation de la tubuline choisi parmi la cytokératine endoA présentant au moins 80%, de préférence 85%, 90%, 98% et 100% d'identité après alignement optimal avec la séquence SEQ ID N°9, la kératine 8 présentant au moins 80%, de préférence 85%, 90%, 98% et 100% d'identité après alignement optimal avec la séquence SEQ ID N°10, la périphérine présentant au moins 80%, de préférence 85%, 90%, 98% et 100% d'identité après alignement optimal avec la séquence SEQ ID N°11, l'internexine présentant au moins 80%, de préférence 85%, 90%, 98% et 100% d'identité après alignement optimal avec la séquence SEQ ID N°12, la GFAP présentant au moins 80%, de préférence 85%, 90%, 98% et 100% d'identité après alignement optimal avec la séquence SEQ ID N°13 ou la vimentine présentant au moins 80%, de préférence 85%, 90%, 98% et 100% d'identité après alignement optimal avec la séquence SEQ ID N°14 pour l'obtention de médicaments.

La présente invention a également pour objet un fragment de filament intermédiaire selon la présente invention pour la mise en évidence et/ou pour tester in vitro des produits susceptibles de bloquer l'interaction entre la tubuline et ledit fragment de filament intermédiaire

Plus particulièrement, un fragment de filament intermédiaire peut être la cytokératine endoA de séquence SEQ ID N°9, la kératine 8 de séquence SEQ ID N°10, la périphérine de séquence SEQ ID N°11, l'α-internexine de séquence SEQ ID N°12, la GFAP de séquence SEQ ID N°13 ou la vimentine de séquence SEQ ID N°14.

Les séquences SEQ ID N°9 et SEQ ID N° 10 sont identiques.

Selon les analyses bioinformatiques, notamment en utilisant le programme Clustal W (cf. Tableau 1), une homologie de séquence existe entre la cytokératine endoA présentant au moins 80%, de préférence 85%, 90%, 98% et 100% d'identité après alignement optimal avec la séquence SED ID N°9, la kératine 8 présentant au moins 80%, de préférence 85%, 90%, 98% et 100% d'identité après alignement optimal avec la séquence SED ID N°10, la périphérine présentant au moins 80%, de préférence 85%, 90%, 98% et 100% d'identité après alignement optimal avec la séquence SED ID N° 11, l'α-internexine présentant au moins 80%, de préférence 85%, 90%, 98% et 100% d'identité après alignement optimal avec la séquence SED ID N°12, la GFAP présentant au moins 80%, de préférence 85%, 90%, 98% et 100% d'identité après alignement optimal avec la séquence SED ID N°13, ou la vimentine présentant au moins 80%, de préférence 85%, 90%, 98% et 100% d'identité après alignement optimal avec la séquence SED ID N°14.

La présente invention sera mieux comprise à l'aide du complément de description qui va suivre, qui se réfère à un exemple montrant que la tubuline et les neurofilaments sont des partenaires de liaison et nous démontrons que cette interaction joue un rôle critique dans la modulation des dynamiques des microtubules neuronaux.

Il va de soi, toutefois que cet exemple n'est donné qu'à titre d'illustration de l'objet de l'invention, dont il ne saurait constituer en aucune manière une limitation.

### Légende des figures

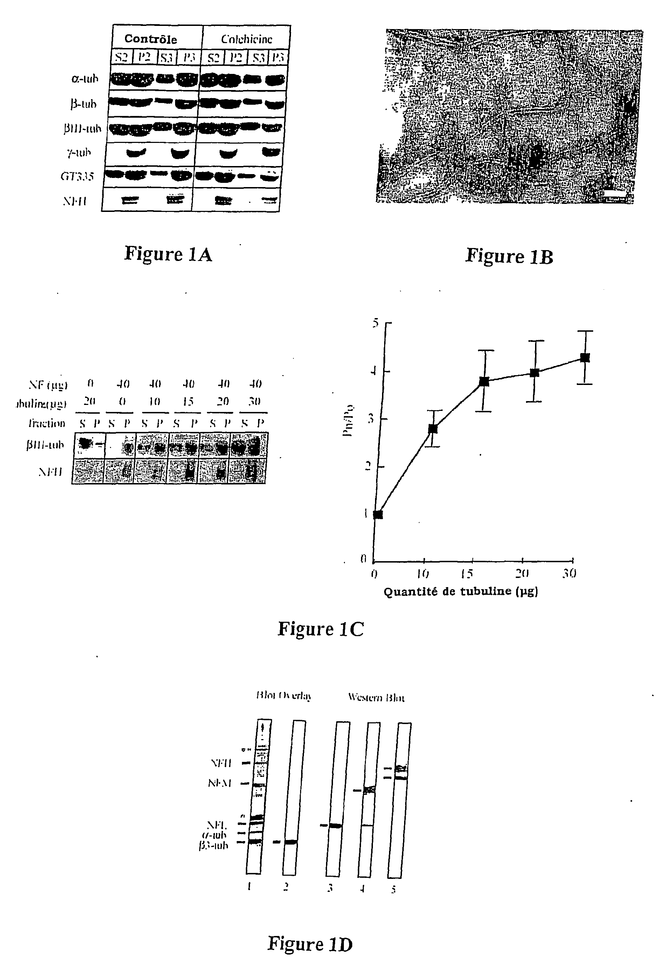

**Figure 1** **: La tubuline co-purifiée avec les neurofilaments à 4°C conserve ses propriétés de polymérisabilité en microtubule et est une protéine associée aux trois sous-unités des neurofilaments.**
   (A) : En utilisant des anticorps dirigés contre les différents isotypes de tubuline (α, β, βIII, γ et polyglutamylée), la tubuline a été détectée dans chacune des fractions d'une préparation classique des neurofilaments. Malgré une incubation à 4°C ou la présence de colchicine, une grande quantité de tubuline est systématiquement co-purifiée avec les neurofilaments.
   (B) : Lorsqu'un culot P3 incubé à 4°C sans colchicine est observé en microscopie électronique, aucun microtubule n'est décelable sur la grille de microscopie (données non montrées). Cependant, lorsque cette préparation est incubée à 37°C en présence de GTP, de nombreux microtubules sont observés avec les neurofilaments. Certains neurofilaments sont décorés par du matériel amorphe qui pourrait être de la tubuline (barre : 100 nm).
   (C) : 40 µg de NF ont été incubés 1 heure en présence de tubuline purifiée puis centrifugée à 100000 g pendant 30 min. Une analyse par Western-Blot des surnageants et culots montre que la tubuline présente dans les culots augmente proportionnellement avec la quantité de tubuline exogène ajoutée pour l'incubation jusqu'à un plateau, ce qui montre que l'interaction NF-tubuline est saturable.
   (D) : Les protéines présentes dans le culot P3 ont été séparées par SDS-PAGE et transférées sur une membrane de PVDF puis incubées avec de la tubuline purifiée sur colonne de phosphocellulose. Les membranes ont ensuite été incubées avec un anticorps anti-βIII tubuline afin de procéder à un Western-Blot classique. Les résultats montrent que la tubuline interagit avec des bandes correspondant à NFL, NFM, et la forme phosphorylée de NFH, l'α- et la β-tubuline, ainsi qu'avec la synapsine* et les MAPs** (1). Lorsqu'une expérience similaire est réalisée en l'absence de tubuline, on n'observe seulement une bande correspondant à la βIII-tubuline (2). La même membrane (1) a été hybridée successivement avec les différents anticorps anti-NFL (3), anti-NFM (4), and anti-NFH (5). L'anticorps anti-NFH reconnaît les deux formes phosphorylées et non phosphorylées de NFH.

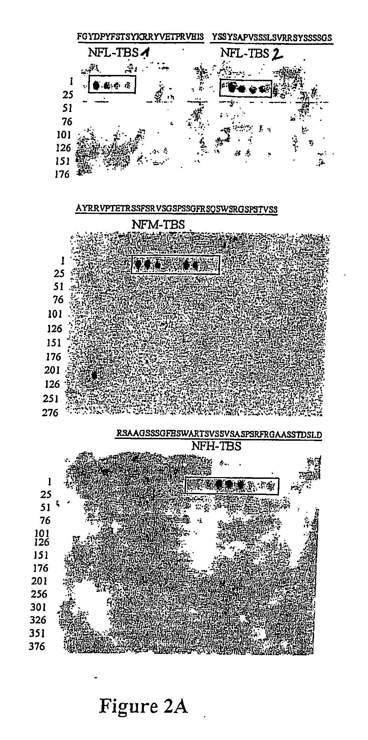

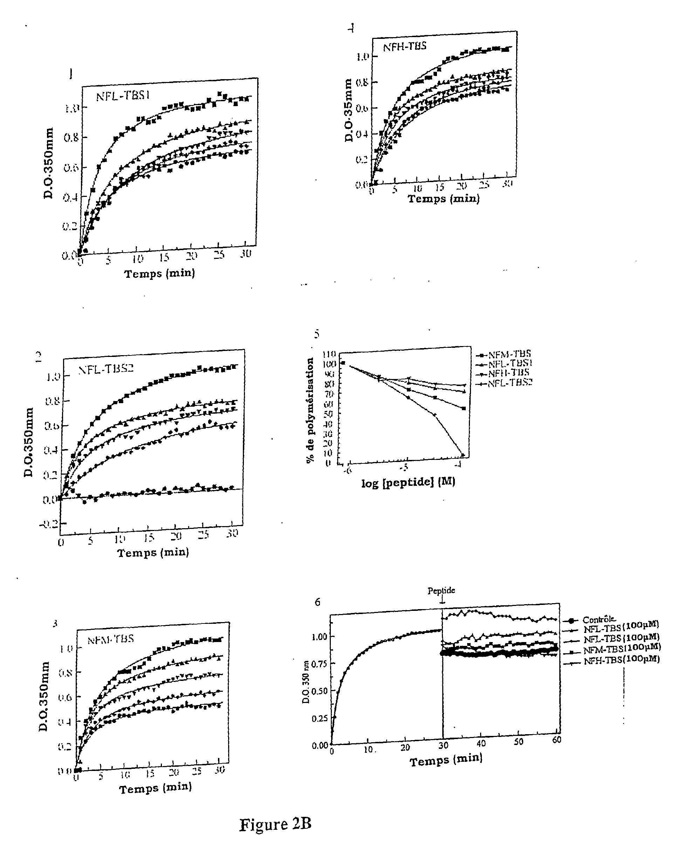

**Figure 2** **: La tubuline se lie sur l'extrémité N-terminale de chacune des sous-unités des neurofilaments. Effet de chacune des séquences correspondant à ce site de liaison sur la polymérisation des microtubules.**
   (A): Les membranes de peptide array ont été incubées comme dans la procédure décrite par Frank et Owerwin (1996) avec de la tubuline purifiée sur colonne de phospho-cellulose toute la nuit à 4°C. La tubuline fixée a été révélée avec un anticorps anti-βIII tubuline et un anticorps secondaire couplé à la peroxydase. La sous-unité NFL contient deux domaines de liaison à la tubuline (nommés NFL-TBS1 and NFL-TBS2), alors que NFM et NFH contiennent une seule séquence de liaison de 39 amino acides (nommées respectivement NFM-TBS et NFH-TBS).
   (B): Chacun de ces peptides correspondant au site de liaison de la tubuline ont été testés in vitro pour leur effet sur la polymérisation de la tubuline en microtubules à différentes concentrations: 100 µM (●), 30 µM (◆), 10 µM (▼), 3 µM (▲) ou sans peptide (■). (1): NFL-TBS1, (2): NFL-TBS2, (3): NFM-TBS and (4): NFH-TBS. (5): courbe de l'effet inhibiteur de chaque peptide en fonction du log de la concentration.
**Figure 3** **: Photos en microscopie électronique de coupes transversales d'axones de souris contrôles et transgéniques.** Les neurofilaments observés dans les axones de souris contrôles sont absents dans les axones de souris transgéniques (barre = 100nm)
**Figure 4****: La quantité d'ARNm d'α-tubuline présente chez les souris contrôles et NFHLacZ est la même.**
   (A): Pendant le développement la quantité d'ARNm de tubuline Mα1 chute d'un facteur 10 aussi bien dans le cerveau que dans la moelle épinière. Un profil d'expression similaire est observé entre les deux génotypes.
   (B): Les taux d'ARNm de la tubuline Mα2 restent constants au cours du développement aussi bien dans le cerveau que dans la moelle épinière sans différence entre contrôles et transgéniques. (-: contrôles, +: transgéniques, 2D: 2 jours, 4w: 4 semaines, ad: adultes, et n représente le nombre de cerveaux et de moelles épinières utilisées pour réaliser les expériences en triplicata.).
   (C) : Des ARNm de la tubuline Mα3/7 ont été détectés seulement dans les testicules. Aucune expression ectopique n'a été détectée.

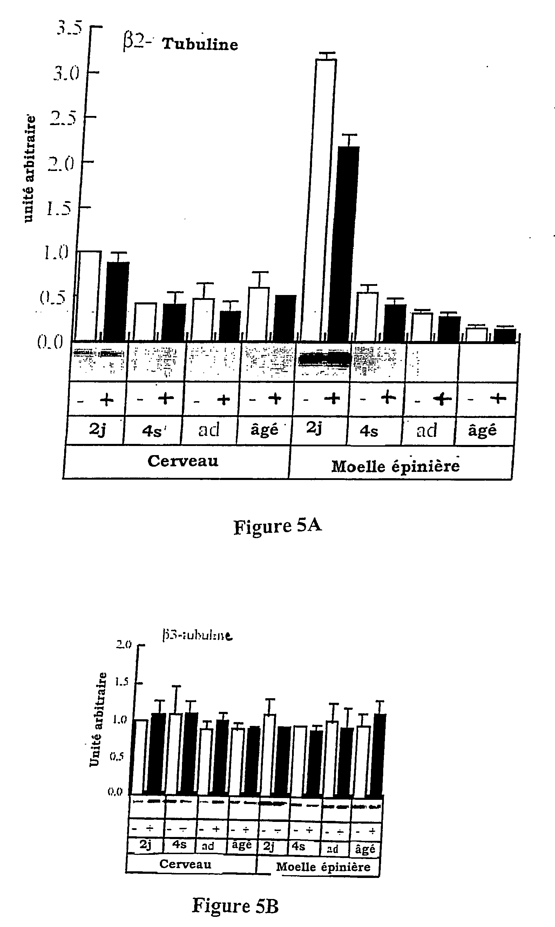

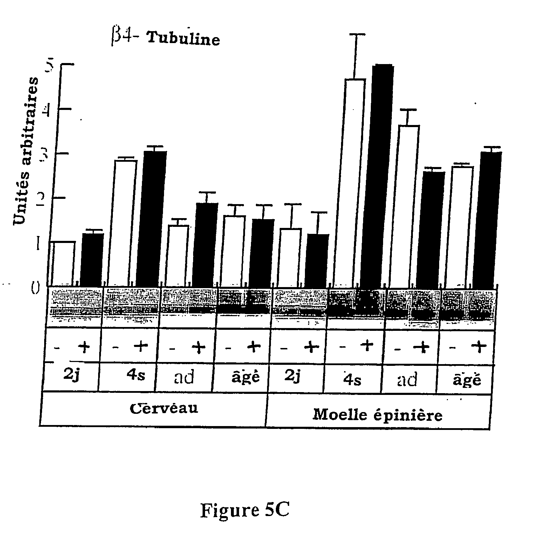

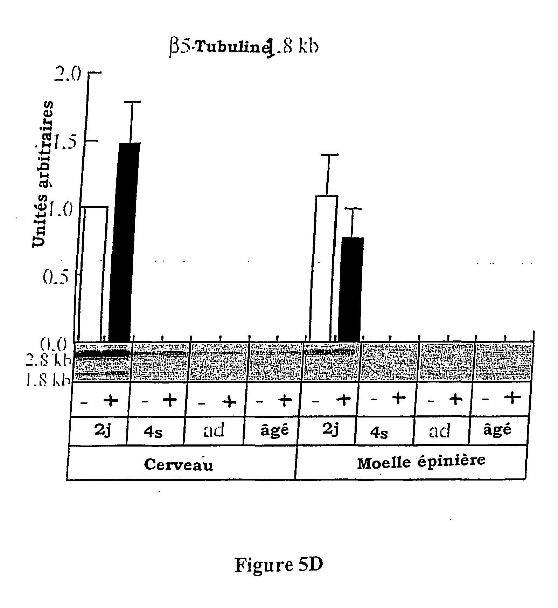

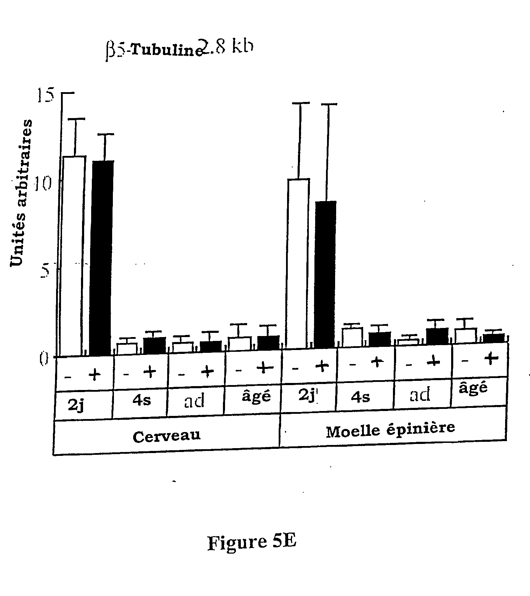

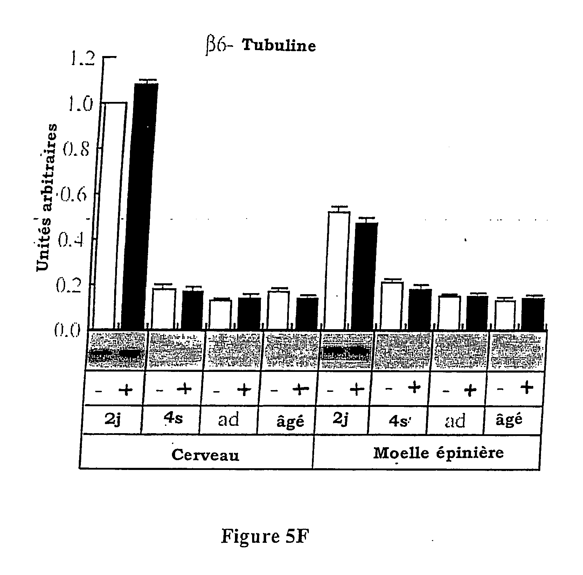

**Figure 5****: La quantité d'ARNm de tubuline β est aussi inchangée entre les souris contrôles (-) et transgéniques (+).**
   (A): La quantité d'ARNm de tubuline Mβ2 diminue aussi bien dans le cerveau (facteur 3) que dans la moelle épinière (facteur 10) sans différence entre les souris contrôles et transgéniques.
   (B) : Des ARNm de tubuline Mβ3 ont été détectés à des niveaux similaires dans les souris transgéniques et contrôles.
   (C): Les ARNm de tubuline Mβ4 ont un profil biphasique dans le cerveau et la moelle épinière. Tout d'abord le taux d'ARNm augmente entre les stades 2 jours et 4 semaines d'un facteur 3 dans le cerveau et 5 dans la moelle épinière. Puis le taux diminue d'un facteur 2 dans le cerveau et la moelle épinière sans différence majeure entre les souris contrôles et transgéniques.
   (D-E): Deux transcripts pour la tubuline Mβ5 ont été identifiés dans le cerveau et la moelle épinière. Leur profil d'expression est similaire entre les souris contrôles et transgéniques. Durant le développement les deux transcrits voient leur quantité diminuer d'un facteur 10. Le transcrit le plus gros (2,8 Kb) est exprimé dix fois plus que le transcrit de 1,8 kb.
   (F): Une diminution d'un facteur 5 dans le cerveau et 3 dans la moelle épinière est observé pour le taux d'ARNm de la tubuline Mβ6. Ce transcrit est surtout observé durant les stades précoces du développement sans différence entre les deux génotypes.

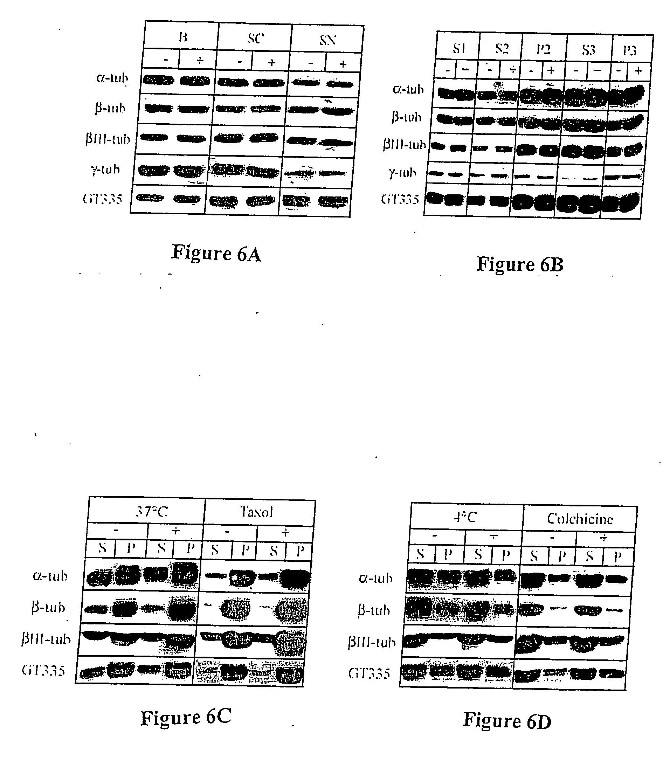

**Figure 6****: La quantité de tubuline présente dans les homogénats tissulaires des souris contrôles et transgéniques n'est pas différente.**
   (A): La quantité de tubuline présente dans les extraits bruts de cerveaux (B), moelle épinière (SC) et nerfs sciatiques (SN) de souris contrôles et transgéniques est la même.
   (B): La quantité de tubuline présente dans les différentes fractions d'une préparation classique de microtubules est identique entre les souris contrôles et transgéniques.
   (C, D): Afin d'évaluer la capacité de polymérisation de la tubuline des souris contrôles et transgéniques des surnageants S1 de cerveaux contrôles et transgéniques on été incubés 1 heure à 37°C en présence de GTP ou en présence de Taxol pour provoquer la polymérisation des MT (C). ou au froid ou en présence de Colchicine pour induire la dépolymérisation des microtubules. (D). Après une centrifugation à 100000 g dans les mêmes conditions, les surnageants et culots on été analysés par Western-Blot a l'aide d'un panel d'anticorps dirigés contre les différentes tubulines. Il n'existe pas de différence entre les souris contrôles et transgéniques.

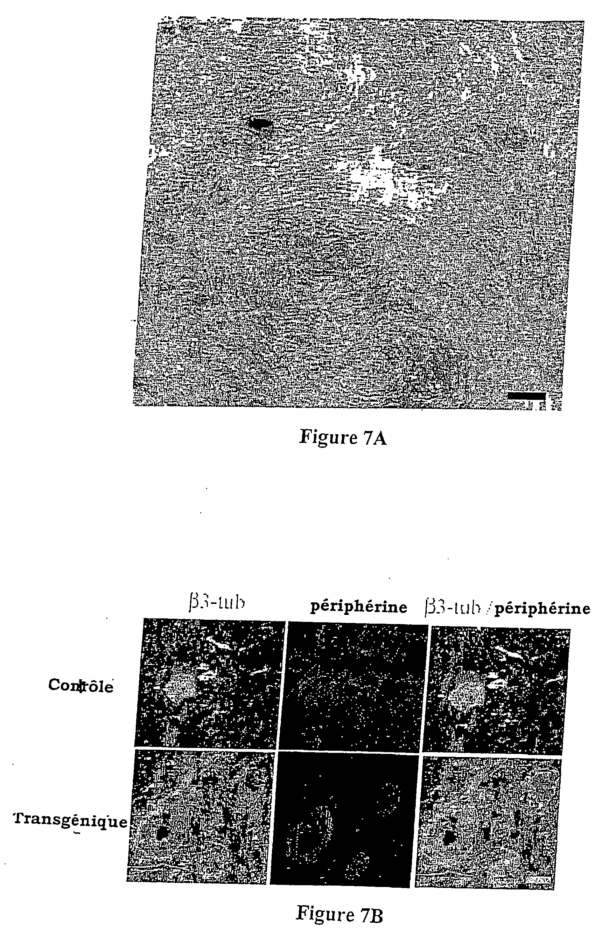

**Figure 7****: En microscopie électronique aucun microtubule n'est observé dans les agrégats de neurofilaments malgré la présence d'épitopes de tubuline mis en évidence par immunofluorescence couplée à la microscopie confocale.**
   (A) : Photos de microscopie électronique d'un agrégat de neurofilaments présent dans le corps cellulaire d'un moto-neurone de souris. Aucun microtubule ne peut être observé. (barre: 200 nm).
   (B) : Analyse immunohistochimique couplée à la microscopie confocale de moelles épinières de souris contrôles et NFHLacZ à l'aide d'anticorps dirigés contre la tubuline βIII et la périphérine. Les deux réseaux de neurofilaments et de microtubules sont co-localisés dans le corps cellulaire des souris contrôles. Malgré l'absence de microtubule dans le corps cellulaire, les agrégats de neurofilaments contiennent des épitopes de tubuline (barre: 12,5 µm).

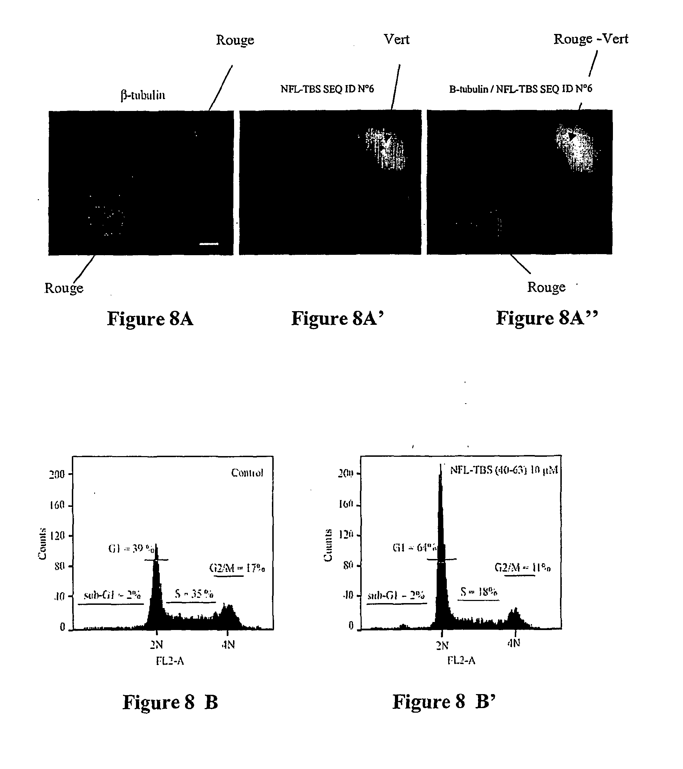

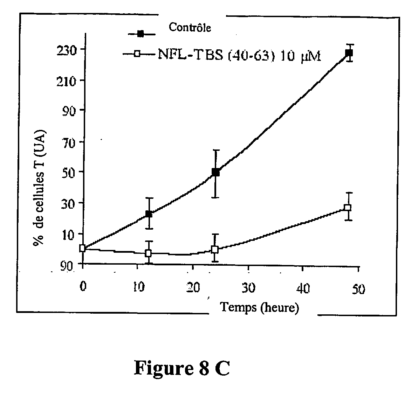

**Figure 8** **: Effet des peptides NFL-TBS (SEQ ID N°6) sur le cytosquelette des microtubules, et sur la division cellulaire.**
   (A-A'-A") : Les cellules T98G sont traitées avec les peptides SEQ ID N°6 (10µM) pendant 6 heures, et les microtubules sont ensuite détectées en utilisant un anticorps anti-tubuline (rouge) et les peptides SEQ ID N°6 biotinylés sont détectés en utilisant de l'avidine marquée à l'Alexa (vert). Les cellules qui contiennent les peptides manquent de réseau typique de microtubule et ont une forme ronde alors que les cellules normales ont un réseau normal de microtubules.
   (B-B') : Analyse en cytométrie en flux des cellules contrôles et des cellules traitées avec les peptides SEQ ID N°6. Les cellules T98G traitées avec les peptides SEQ ID N°6 (10 µM) pendant 48 heures montrent un arrêt fort en phase G1 comparé aux cellules contrôles.
   (C) : Essai de test de prolifération de cellules T98G avec les peptides SEQ ID N°6 : la prolifération de cellules traitées avec le peptide (10 µM pour 48 heures) est fortement réduite par rapport aux cellules contrôles non traitées.
**Figure 9** **(A, B, C, D) :**
   En utilisant la même approche expérimentale que celle décrite pour les puces peptidiques de neurofilament, les puces peptidiques correspondant à la séquence d'autres filaments intermédiaires (**A.** desmine, **B.** vimentine, **C.** cytokératine et **D.** GFAP) ont été testées pour mettre en évidence la présence éventuelle de site de liaison à la tubuline. Chaque filament intermédiaire contient trois sites de liaison à la tubuline (alignement de 2 points ou plus). Deux sites sont localisés dans le domaine N-terminal et un dans le domaine C-terminal.
**Figure 10** **(A, B, C, D, E) :**
   Influence des peptides Vim-TBS3 (SEQ ID NO : 22), GFAP-TBS3 (SEQ ID NO : 28), Ker-TBS1 (SEQ ID NO : 23), Vim-TBS2 (SEQ ID NO : 21) et GFAP-TBS 1 (SEQ ID NO : 26) sur la polymérisation des microtubules.
   En utilisant la même approche expérimentale que celle décrite pour les peptides TBS issus des neurofilaments, l'influence sur la polymérisation des microtubules a été testée pour **A.** Vim-TBS3 (SEQ ID NO : 22) **B.** GFAP-TBS3 (SEQ ID NO : 28) .C. Ker-TBS1 (SEQ ID NO : 23) **D.** Vim-TBS2 (SEQ ID NO : 21) et **E.** GFAP-TBS1 (SEQ ID NO : 26)
**Figure 11** **:**
   Effet du peptide GFAP-TBS1 (SEQ ID NO : 26) sur la division cellulaire. Analyse en cytométrie en flux des cellules contrôles et des cellules traitées avec le peptide GFAP-TBS1 (SEQ ID NO : 26). Les cellules T98G traitées avec le peptide GFAP-TBS1 (SEQ ID NO : 26) (100µM) pendant 36 heures montrent un arrêt fort en phase G1 comparé aux cellules contrôles.

### Exemples

### ► Exemple 1.

### I Introduction

Nous fournissons ici une preuve in vitro et in vivo que la tubuline et les neurofilaments sont des partenaires de liaison et nous démontrons que cette interaction joue un rôle critique dans la modulation de la dynamique des microtubules neuronaux. Tout d'abord en appliquant des séries de techniques biochimiques classiques, nous avons montré que la tubuline non polymérisée co-purifie avec les neurofilaments assemblés, et interagit directement avec les trois sous unités de neurofilaments. Nous avons ensuite utilisé un microscope électronique pour montrer que la tubuline non polymérisée associée avec les neurofilaments garde une capacité totale de s'assembler en microtubules. Pour étudier l'étendue et la signification in vivo de cette association, nous avons utilisé des souris transgéniques exprimant le transgène NFHLacZ. Ces souris expriment de faibles niveaux de protéines de fusion NFH-β-galactosidase qui réticule les neurofilaments causant la séquestration du cytosquelette de neurofilaments dans des agrégats périkaryaux. Nous avons démontré ici que ni la présence d'agrégats périkaryaux, ni l'absence de neurofilaments dans les axones de souris transgéniques NFHLacZ (Eyer et Peterson, 1994) n'affectent l'accumulation d'ARNm des différents isotypes de tubulines. De façon similaire, ni la quantité totale de protéine de tubuline récupérée à partir des axones ni son potentiel de polymérisation n'ont été altérés dans de tels neurones. Cependant, les axones déficients en neurofilaments ont une croissance radiale atténuée atteignant seulement un calibre de 50% par rapport à la normale et en conséquence leur concentration en tubuline relative est atypiquement forte. En cohérence avec leur forte concentration en tubuline, le nombre et la densité de microtubules dans de tels axones sont supérieurs à la normale. Cependant, le nombre absolu et la densité absolue de microtubules excèdent de beaucoup ce qui a été prédit par l'augmentation de la concentration en tubuline seule et définit donc un rôle majeur pour les neurofilaments dans la régulation de la dynamique des microtubules axonaux. A l'inverse, à l'intérieur des agrégats de neurofilaments denses qui se forment dans le perikaryon neuronal de souris NFHLacz, les épitopes de tubuline sont présents mais les profils de microtubules n'ont pas pu être identifiés. Ces observations combinées démontrent que les neurofilaments et la tubuline non polymérisée sont les partenaires de liaison majoritaires et que cette interaction affecte les dynamiques in vivo des microtubules. Une telle intégration du cytosquelette peut jouer un rôle dans la sculpture de la morphologie axonale avec des effets conséquents dans la fonction de fibres tant en conditions physiologique que pathologique.

### II Matériel et méthode

### 1. Analyse informatique

Les alignements de séquence ont été effectués avec ClustalW et corrigés manuellement. Le domaine central très conservé de chaque filament intermédiaire a été utilisé pour l'ancrage de l'alignement. Le tableau 1 a été effectué avec le logiciel Genedoc.

### 2. Analyse des ARNm par Northern-blot

Les cerveaux et moelles épinières ont été prélevés sur des souris transgéniques NFHlacZ et des souris contrôles, de 2 jours, 4 semaines, 6 mois et 18 mois puis immédiatement congelés dans de l'azote liquide et stockés à -80°C. les ARN totaux ont été extraits selon la méthode de Chomczynski et Sacchi (1987). 10µg d'ARN de chaque échantillon ont été utilisés pour effectuer une électrophorèse sur un gel à 1,2% d'agarose, contenant 2,2 M de Formaldéhyde, et transférés par aspiration sur une membrane en Nylon Nytran N (Schleicher et Schuell). Les ARN ont été liés de façon covalente à la membrane par une exposition aux UV. Les membranes ont été préhybridées avec le tampon « Quick-Hyb » (Stratagene) pendant 20 minutes à 68°C, puis hybridées pendant une heure à 68°C avec des sondes d'ADNc, préalablement marquées par amorçage aléatoire avec ^{α32}P-dCTP (Amersham, 3000 Ci/mmole), puis lavées à température ambiante dans 2x SSC, 0.1%SDS (2 x 15 minutes), et à 60°C dans 0.1x SSC, 0.1% SDS (3 x 15 minutes). Le Northern blot a été quantifié en utilisant un Phosphorimager (Molecular Dynamics) puis exposé à des films autoradiographiques (Biomax MR ;Kodak) à -80°C.

Un ADNc de 1,4 kb coupé par EcoRI et HindIII du gène de la GAPDH (Fort et al., 1985) et un de 0.5 kb coupé par EcoRI et HindIII du gène de HSS-26 (Vincent et al., 1993) ont été utilisés comme sondes de contrôle. Pour analyser l'encodage des ARNm de chaque isotype de tubuline (Lewis et al., 1985; Villasante et al., 1986; Wang et al., 1986), des sondes spécifiques ont été générées à partir des séquences 3' non traduites dans le vecteur pUC par PCR utilisant les primers pUC (5'-3' CTGCAAGGCGATTAAGTTGG et GTTGTGTGGAATTGTGAGCG).

L'intensité relative des bandes a été mesurée en utilisant un Phosphorimager (Molecular Dynamics). Les ARNm isolés à partir des cerveaux des animaux contrôles de 2 jours ont été utilisés comme référence et définis arbitrairement comme 1. Des exceptions ont été faites pour les ARNm de tubuline α3/7 et β1 isolés des testicules et de la rate des souris adultes contrôles, à cause de leur absence d'expression dans les autres tissus. Les valeurs de GAPDH ne montrent pas de variation d'expression entre les valeurs des animaux contrôles et des animaux transgéniques (Robert et al., 2001). De plus, pour chaque Northern blot, des facteurs de correction qui égalisent les valeurs de GAPDH ont été calculés pour normaliser les niveaux d'ARNm. Les valeurs moyennes des différentes expériences à chaque étape post-natale ont été calculées et exprimées en tant que moyenne +/- l'erreur standard.

### 3. Isolation des microtubules, de la tubuline et des neurofilaments.

Les cerveaux, moelles épinières ou nerfs sciatiques ont été disséqués à partir de souris adultes, pesés et homogénéisés dans du tampon RB (mM MES, pH 6,8, 1 mM EGTA et 1 mM MgCl₂). Les homogénats ont été centrifugés à 10⁵g à 4°C pendant 1 heure dans une ultracentrifugeuse Beckman LE-80k ou TLX100. Pour l'isolation des microtubules, le premier surnageant (S1) a été incubé avec 4M de glycérol et 0,1 mM de GTP à 37°C pendant 1 heure, puis centrifugé à 10⁵g à 37°C pour récupérer les microtubules polymérisés et les neurofilaments dans le deuxième culot de centrifugation (P2).

Les microtubules et les neurofilaments présents dans le culot (P2) ont été suspendus dans du tampon RB, incubés à 4°C pendant 1 heure pour dépolymériser les microtubules et centrifugés à 10⁵g à 4°C pendant 1 heure. La fraction de tubuline non polymérisée a été récupérée dans le troisième surnageant (S3) par passage sur une colonne de phosphocellulose comme décrit par Weingarten et al. 1975. Pour la purification des neurofilaments, un protocole similaire a été utilisé, mais le GTP n'a pas été additionné, et toutes les étapes ont été réalisées à 4°C pour prévenir la polymérisation en microtubule, comme précédemment décrit par Leterrier et Eyer (1987).

### 4. Quantification de protéines et analyse par Western-blot.

Le kit de dosage BCA (Pierce) a été utilisé pour quantifier les protéines de chaque fraction. Les protéines ont été séparées de façon subséquente par un gel SDS-PAGE à 7,5% selon Laemmli, (1970), et révélé par du bleu de Coomassie. De manière alternative, les protéines séparées dans des gels similaires ont été transférées sur des membranes de nitrocellulose (Immobilon, Millipore). Pour des analyses d'immunodétection (Towbin et al., 1979) les sous-unités de neurofilaments ont été identifiées en utilisant des anticorps monoclonaux anti-NFH, anti-NFM et anti-NFL (Sigma N5389, N5264, N5139), et un anticorps polyclonal anti-NFH (Sigma N4142). Les isotypes de tubuline ont été identifiés en utilisant des anticorps reconnaissant l'α-tubuline, la β-tubuline, la tubuline βIII-spécifiquement neuronale, la γ-tubuline (Sigma T9026, T4026, T8660) et des épitopes de tubuline poly-glutamylés (GT335).

Les expériences ont été effectuées en triplicata et toutes les membranes ont été hybridées avec chaque anticorps, et déshybridées entre chaque hybridation, selon les instructions du fournisseur. Chaque réaction a été révélée en utilisant un protocole chimioluminescent (ECL, Amersham). Pour évaluer l'intensité de la réaction, les films exposés ont été scannés et quantifiés en mesurant la surface et la densité de chaque spot avec le logiciel ImageQuant (Molecular Dynamics) ou NIHImage. Pour chaque expérience, le signal de l'échantillon de contrôle a été utilisé comme référence.

### 5. Précipitation de la tubuline avec les neurofilaments

Une quantité égale de neurofilaments (40µg) a été incubée avec différentes quantités de tubuline purifiée pendant 1 heure à 4°C puis centrifugée pendant 30 minutes à 100000g à la même température. Le surnageant et les culots resuspendus (dans du RB) ont été stockés à -20°C avant l'analyse par Western-Blot.

### 6. Puce peptidique sur membrane et peptides synthétiques.

Des puces peptidiques correspondant aux sous unités de neurofilaments NFL, NFM et NFH ont été obtenues selon Frank et al., 1992, avec un robot ASP 222 automat spot. Chaque séquence de neurofilament a été divisée en séquences de 15 acides aminés.

Ces peptides chevauchent 12 acides aminés du peptide suivant. Les peptides chevauchants ont été synthétisés sur des spots, comme une puce, sur une membrane de cellulose spécialement produite (AIMS scientific product, GmbH, Braunschweig, Allemagne). Les peptides sont liés à la membrane avec un pont polyéthylène glycol grâce à leur acide aminé carboxy-terminal. La procédure de synthèse suivie est celle de Frank et al. 1996.

### 7. Expériences de blot overlay

Les protéines présentent dans le troisième culot (P3) d'une préparation de microtubules typique (qui est enrichie en neurofilaments), ont été séparées sur un gel SDS-Page à 7,5%, puis transférées sur membrane de nitrocellulose, selon Towbin et al. (1979). Les membranes ont été coupées en bandes, et bloquées avec du TBS (1x) contenant 10% de lait en poudre. Ces bandes ont été incubées une nuit à 4°C avec la tubuline purifiée. Après un lavage extensif avec du TBS (1x) les membranes ont été immuno-marquées avec des anticorps anti-βIII-tubuline ou anti-α-tubuline afin de révéler les protéines qui interagissent avec la tubuline. Les mêmes membranes ont été ensuite deshybridées et re-hybridées avec de nouveaux anticorps afin de confirmer la nature des bandes qui ont été reconnues par la réaction à la tubuline et à l'anti-tubuline.

De manière alternative, les bandes qui ont été révélées ont été découpées et analysées par spectrométrie de masse. La puce peptidique sur membrane a été incubée une nuit à 4°C avec la tubuline purifiée. Après un lavage extensif avec du TBS (1x), les membranes ont été incubées avec des anticorps anti-βIII-tubuline afin de révéler les séquences d'acides aminés qui interagissent avec la tubuline.

### 8. Analyse par spectrophotométrie de la polymérisation des microtubules.

La polymérisation de la tubuline à partir du surnageant S3 d'une préparation de microtubules a été étudiée par turbidimétrie. Brièvement, le surnageant S3 (3mg/ml) a été incubé à 37°C en présence de 1 mM de GTP dans un spectrophotomètre thermospectronique Helios α pour suivre les variations de densité optique à 350 nm.

### 9. Analyses immunohistochimiques en microscopie confocale.

Les souris ont été sacrifiées par injection d'une dose létale d'anesthésique avec de l'avertine (8mg/kg), puis perfusées de façon transcardiaque dans un premier temps avec 50 ml de tampon phosphate afin d'éliminer le sang, suivi de 50 ml de 4% de paraformaldéhyde dans du tampon phosphate.

Les échantillons ont été récupérés, post-fixés dans le même tampon de fixation pendant une heure, puis transférés dans une solution à 30% de sucrose avant d'être congelés dans l'isopentane à -40°C et stockés à -80°C. Les sections de cryostat (10µm) ont été préparées, placées sur des lames et stockées à -80°C pour des analyses immunohistochimiques. Les lames ont été ramenées à température ambiante et rincées 3 fois avec du tampon phosphate avant d'être bloquées à température ambiante pendant 2 heures avec 5% de sérum albumine bovine (BSA) pour les anticorps anti-neurofilaments, ou 5% BSA plus 5% de sérum de chèvre pour les anticorps anti-tubuline. Les sections ont été rincées trois fois, 5 minutes chacune avec du tampon phosphate et incubées une nuit avec les anticorps primaires (dans 1 % de tampon phosphate BSA).

Les sections ont ensuite été rincées trois fois 5 minutes chacune et incubées avec des anticorps secondaires fluorescents (Alexa 488 nm anticorps anti-souris, et Alexa 568 nm anticorps anti-lapin de Molecular Probes, dilué au 1/200). Chaque anticorps a été incubé consécutivement une heure et demie puis rincées trois fois pendant 5 minutes.

Les lames ont été fixées avec un milieu anti-vieillissement et stockées à 4°C dans l'obscurité avant observation. Les anticorps primaires et les dilutions ont été utilisés comme ce qui suit : les anticorps monoclonaux anti-α-tubuline, anti-β-tubuline et anti-βIII-tubuline ont été dilués à 1:100; les anticorps polyclonaux anti-NFH et anti-périphérine ont été dilués à 1:1000 et 1/100 respectivement. Les lames immunomarquées ont été analysées avec un microscope confocal Olympus (BX50) utilisant le logiciel Olympus Fluoview.3.0.

### 10. Microscopie électronique et quantification.

L'analyse par microscope électronique a été effectuée selon les protocoles précédemment décrits (Eyer et Peterson, 1994). La densité des microtubules axonaux a été évaluée en scannant les négatifs de micrographie électronique (pris à 25000x en utilisant un microscope électronique JEOL JEM2010) des axones sélectionnés de manière aléatoire de chaque génotype en utilisant un scanner U-MAX/ASTRA2400S. Les images ont été importées sur un ordinateur 8600 PowerPC/Macintosh utilisant Photoshop. La densité de microtubules a été évaluée en comptant le nombre de microtubules/hexagone comme décrit par De Waegh et al., 1992.

### III Résultats

### 1 La tubuline non polymérisée est co-isolée avec les neurofilaments (Figure 1).

Des neurofilaments sont isolés de cerveaux ou de moelles épinières de souris normales par des procédures biochimiques classiques.

La purification initiale implique l'adjonction de glycérol 4M fmal au surnageant (S1) obtenu à partir de l'homogénat brut.

Quand ces mélanges sont incubés pendant une heure à une température de 4°C, les neurofilaments forment un gel qui se dépose dans le second culot (P2) après centrifugation (Leterrier & Eyer, 1987).

Quand la phase d'incubation / centrifugation est répétée avec le culot P2, d'autres neurofilaments peuvent être récupérés dans le culot P3. Etrangement, l'analyse par Western Blot des fractions enrichies en neurofilaments révèle la présence d'espèces de tubulin à la fois α et β tout autant que de γ. Des résultats similaires ont été obtenus quand les surnageants étaient incubés avec de la colchicine (jusqu'à 3mM), qui empêche l'assemblage de microtubules, ou après de multiples cycles de sédimentation et de remise en suspension des dépôts à basse température, une condition qui empêche aussi l'assemblage de microtubules (Figure 1A).

L'examen des neurofilaments récupérés dans les fractions P3 par microscopie électronique a révélé une abondance de neurofilaments hautement décorés par du matériel amorphe. Au contraire, les microtubules dans ces fractions étaient rares et quand on les trouvait, courts. Typiquement, quand 10µg de la fraction P3 à une température de 4°C était chargée sur une grille à maillage de 300, seuls quelques rares carrés de grille contenaient des microtubules courts. Cependant, quand ces mêmes fractions étaient incubées à une température de 37°C en présence de 1mM GTP, pour favoriser la polymérisation, beaucoup de microtubules longs étaient reconnaissables dans chaque carré de grille (jusqu'à 50) et ces derniers étaient souvent associés à des neurofilaments (figure 1B).

Ces observations démontrent que la tubuline non polymérisée (isolation froide) est associée aux neurofilaments. Cette tubuline conserve tout son potentiel de polymérisabilité en conditions favorables (37°C en présence de GTP).

Afin de comparer la quantité relative de tubuline sédimentant en microtubules à celle liée aux neurofilaments, comme démontrée par la sédimentation dans la fraction de neurofilaments, l'échantillon brut de cerveau (S1) a été incubé en présence de GTP et de glycérol, soit à 37°C soit à 4°C, avant centrifugation à la même température.

Après incubation à 37°C, 9,55 ± 1,14% des protéines totales sont retrouvées dans le culot alors que 90,45 ± 1,14% est restée dans le surnageant.

Après incubation à 4°C, 4,53 ±0,34% est présent dans le culot alors que 95,43 ± 0,34% est resté dans le surnageant (n=3).

Il apparaît donc que 5% des protéines totales contenues dans l'échantillon brut (S1) représente la tubuline non polymérisée capable, avec des protéines associées, de polymériser en microtubules.

Afin d'évaluer la capacité des neurofilaments à lier la tubuline, une même quantité de neurofilaments a été incubée avec des quantités croissantes de tubuline pendant une heure à 4°C, puis centrifugée pendant 30 minutes à 100000g.

L'analyse par Western-Blot de chaque culot et des surnageants a ensuite été effectuée par Western blot pour déterminer leur contenu en tubuline.

La Figure 1C montre que la tubuline présente dans le culot avec les neurofilaments a augmenté quand la tubuline exogène a été rajoutée de manière saturable, suggérant ainsi une interaction saturable entre les deux partenaires. Remarquons que, sans neurofilament, la tubuline présente dans le dépôt est beaucoup plus faible que la quantité de tubuline dans le dépôt sans tubuline exogène ajoutée.

### 2 Identification des séquences de liaison de la tubuline sur les neurofilaments et étude de l'effet des séquences in vitro (Figure 1D, Figure 2 et Tableau 1).

Afin de pousser plus avant l'examen des partenaires moléculaires de cette interaction, nous avons procédé aux expériences de recouvrement de Blot ou blot overlay (Figure 1D).

La troisième fraction d'une préparation de neurofilaments (P3), qui est enrichie en neurofilaments, a été séparée sur un gel à 7,5% SDS-PAGE, puis transférée sur une membrane de nitrocellulose. La membrane a été incubée avec de la tubuline pure, a suivi ensuite le traditionnel Western Blot utilisant des anticorps anti-βIII-tubuline pour identifier les protéines qui interagissent avec la tubuline (figure 1D ligne 1). Cette approche a révélé une interaction directe entre la tubuline et les trois sous-unités des neurofilaments NFL (3), NFM (4), et NFH (5).

Quand on ne procède pas à l'ajout de tubuline, la tubuline anti-βIII ne reconnaît qu'un seul groupe, la tubuline βIII qui est présente dans la fraction P3 (2). Considérant, l'intensité des signaux, l'interaction de la tubuline est plus intense avec NFL et NFM qu'avec NFH.

De plus, seule l'isoforme phosphorylée de NFH lie la tubuline. Deux signaux additionnels ont été détectés avec cette méthode et correspondent à la synapsine I et II et aux protéines MAPs.

Cependant, la synapsine n'a pas été co-purifiée avec les neurofilaments et ne s'est pas co-agrégée avec les neurofilaments dans les corps cellulaires des souris NFHLacZ (données non communiquées, voir ci-dessous)

Afin d'identifier les séquences responsables de la liaison de la tubuline aux neurofilaments, nous utilisons des puces peptidiques sur membrane et correspondant aux trois sous unités de neurofilaments (voir procédures d'expérimentation). Cette méthode permet l'identification du domaine liant impliqué dans l'interaction protéine-protéine. L'expérience de Blot overlay avec de la tubuline purifiée sur les puces peptidiques a révélé que le site liant la tubuline est situé dans le domaine N-terminal des séquences de neurofilaments. La sous-unité NFL contient deux séquences liantes de tubuline de vingt quatre aminoacides que nous appelons NFL-TBS1 et NFL-TBS2 alors que NFM-TBS et NFH-TBS contiennent tous deux trente neuf aminoacides (Figure 2A).

Nous avons précédemment démontré dans la figure 1 que les neurofilaments renfermaient de la tubuline non polymérisée. Considérant ce résultat, l'identification des sites liant la tubuline peut avoir une pertinence fonctionnelle in vitro sur la polymérisation de la tubuline, c'est à dire peut être capable d'influencer la dynamique microtubulaire. Afin de tester cette hypothèse, nous avons pratiqué des expériences en turbidimétrie avec les différentes séquences de peptide identifiées par blot overlay sur puce peptidique. Dans ce but, le surnageant S3 (contenant de la tubuline libre), provenant d'une préparation de microtubule, a été polymérisée à 37°C en présence de 1mM GTP et de plusieurs concentrations (3, 10, 30, et 100 µM) de peptide liant la tubuline, et qui ont été ajoutés avant la polymérisation (t=0). La figure 2C, 1, 2, 3, 4 montre que chaque peptide a un effet d'inhibition dose-dépendant sur la formation des microtubules. La capacité d'inhibition des peptides était approximativement de 30% avec une concentration de 100 µM sauf pour NFL-TBS2 qui inhibe à 100% la polymérisation de la tubuline à 100 µM (Figure 2B 5). Il semble que le processus impliqué dans cette inhibition soit spécifique au mécanisme de polymérisation puisque ces peptides sont sans effet sur les microtubules préformés, à 30 minutes de polymérisation (figure 2B 6).

Des analyses bioinformatiques sur les séquences N-terminales des neurofilaments montrent que les peptides testés ci-dessous sont positionnés dans une zone hautement conservée de ce domaine si on considère le nombre d'aminoacides conservés entre les trois sous-unités de neurofilaments et entre les espèces (BLAST). Cette conservation est spécifique au domaine N-Terminal des neurofilaments (Tableau 1) et semble être perdue ou moins forte quand on pratique un alignement avec d'autres types de filaments intermédiaires.

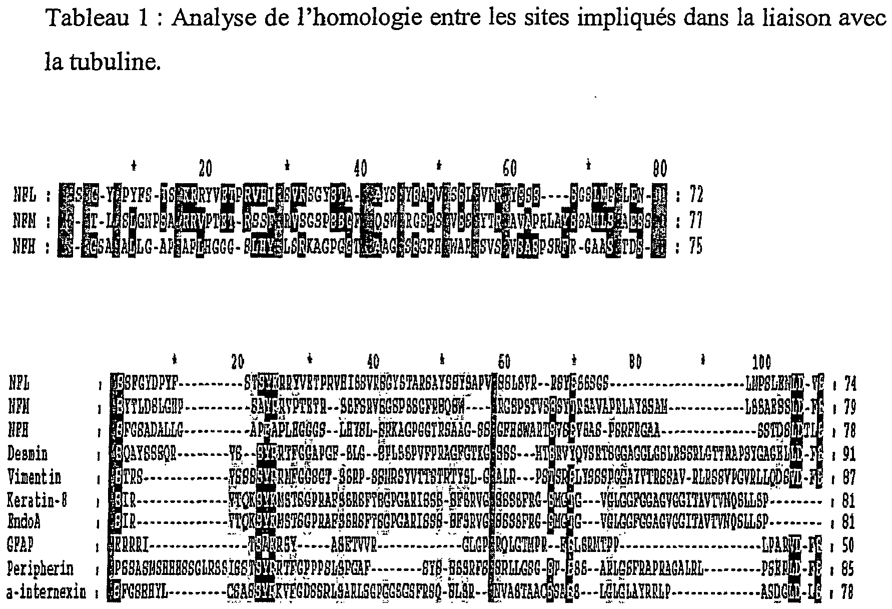

La séquence des trois sous-unités des neurofilaments et de divers filaments intermédiaires ont été alignées avec le programme ClustalW en fonction de leur homologie (endoA : cytokératine endoA; Ker 8 : Kératine 8; NFL : sous-unité légère des neurofilaments; NFM : sous-unité moyenne des neurofilaments; NFH : sous-unité lourde des neurofilaments; periph : périphérine; internex : α-intemexine; gfap : gliofilament; vim : vimentine)

De nombreux acides aminés sont conservés dans la partie N-terminale des trois sous-unités des neurofilaments et dans une moindre mesure dans les domaines N-terminaux des autres filaments intermédiaires.

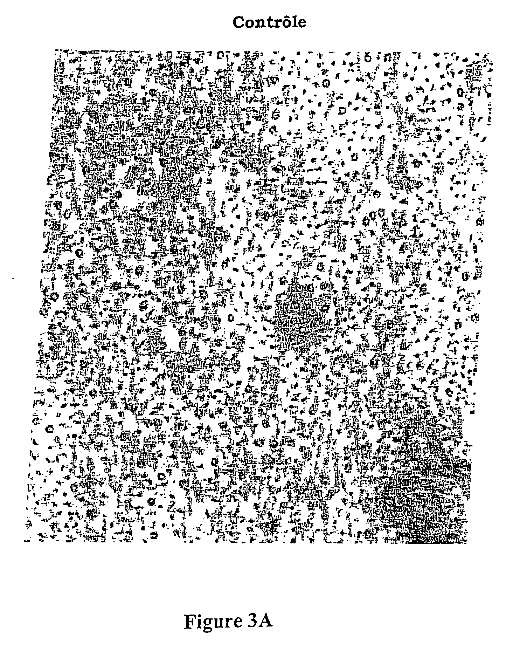

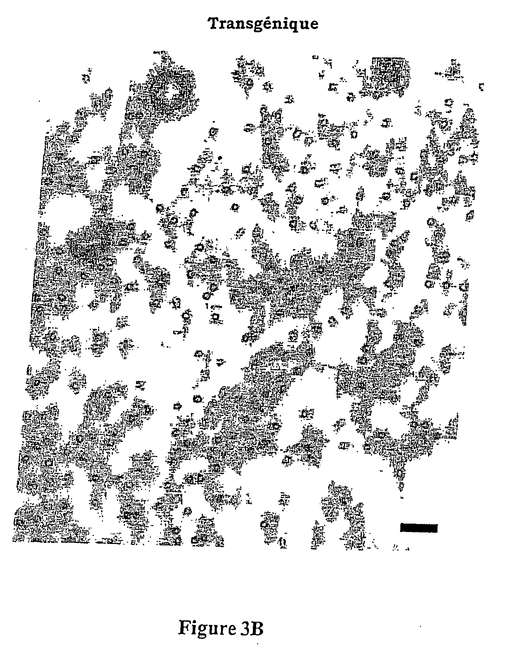

### 3 Analyse morphométrique du cytosquelette microtubulaire axonal chez les souris transgéniques NFHlacZ (figure 3)

Afin d'étudier la signification in vivo de l'association neurofilament - tubuline mise en évidence dans les recherches biochimiques ci dessus, nous avons étudié des souris transgéniques NFHlacZ chez lesquelles la répartition des neurofilaments est perturbée.

Sous l'influence des éléments régulateurs de NFH, le transgène NFHlacZ est exprimé dans la plupart des neurones.

La protéine de fusion codée par le transgène s'assemble au réseau de neurofilaments, et, à travers les domaines de dimérisation de la β-galactosidase, réticule les neurofilaments assemblés, et prévient leur export vers le compartiment axonal. En dépit de la mauvaise répartition du cytosquelette des neurofilaments, ces neurones sont généralement viables jusqu'à un âge avancé. (Eyer & Peterson, 1994).

Afin de déterminer si l'absence de neurofilaments dans les axones des souris transgéniques NFHlacZ affecte le cytosquelette microtubulaire, des échantillons de nerfs sciatiques et de moelles épinières provenant de souris âgées ont été examinés par microscopie électronique (Figure 3). Un réseau caractéristique et dense de neurofilaments a été observé dans les coupes de tous les axones de gros calibre provenant des nerfs sciatiques normaux.

Chez les échantillons transgéniques, peu si ce n'est aucun neurofilaments ne pouvait être reconnu, alors qu'au contraire, le cytosquelette microtubulaire axonal était particulièrement dense.

Afin d'évaluer la densité microtubulaire, les profils de microtubule rencontrés à l'intérieur d'une superficie de coupe prédéterminée ont été comptabilisés. (De Waegh et al., 1992). Les axones témoins contenaient 9,44 ± 1,4 microtubules/µm² alors que dans la même zone axonale chez les échantillons transgéniques (nombre d'axones = 500), la densité microtubulaire était plus de 10 fois supérieure à 99,06 ± 5,1 microtubules/µm².

Il a été précédemment démontré que le diamètre moyen des axones chez les souris transgéniques NFHlacZ était réduit de 50% (Eyer & Peterson, 1994). Donc, la densité microtubulaire élevée dans les axones pourrait représenter la simple conséquence de la baisse de volume axonal. Cependant, les changements de volume seuls ne suffisent pas à expliquer l'ampleur de l'augmentation de densité tubulaire observée, c'est à dire à assumer un profil circulaire, un axone normal ayant un diamètre de 10µm avec une superficie de coupe de 78,5 µm², et à 9,44 ± 1,4 microtubules/µm² devrait contenir environ 741 profils microtubulaires.

Si le même axone était déficient des neurofilaments, son diamètre serait réduit à 5 µm et aurait une superficie de coupe de 19,6 µm². Si le même nombre de microtubules étaient présents, leur densité serait de 741/19,6µm² ou 37,8 microtubules/µm².

Donc une augmentation de densité maximale de quatre fois (37,8/9,44 = 4,0) pourrait être due à la seule réduction du calibre axonal de 50%. En conséquence, les échantillons transgéniques contiennent deux fois et demi plus de microtubules que le laissaient prévoir les seuls paramètres du volume et de la concentration puisque la densité microtubulaire est augmentée de dix fois.

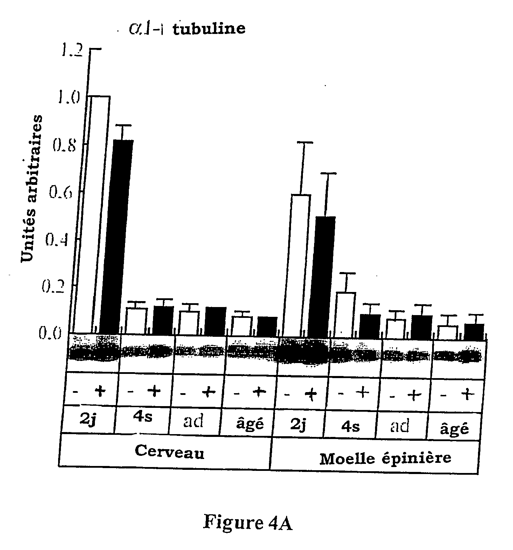

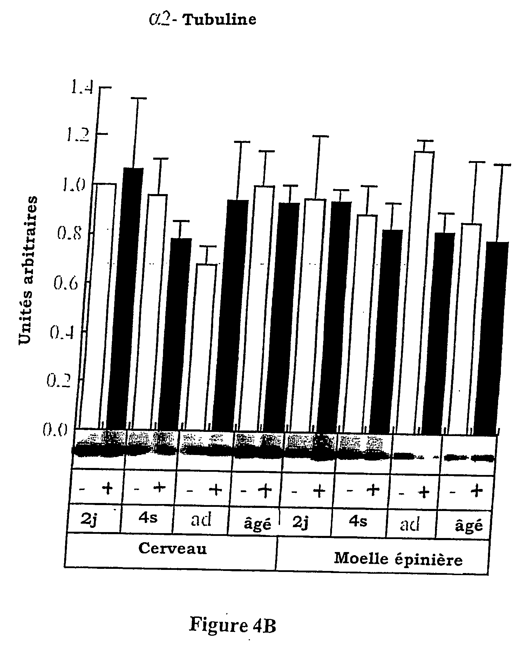

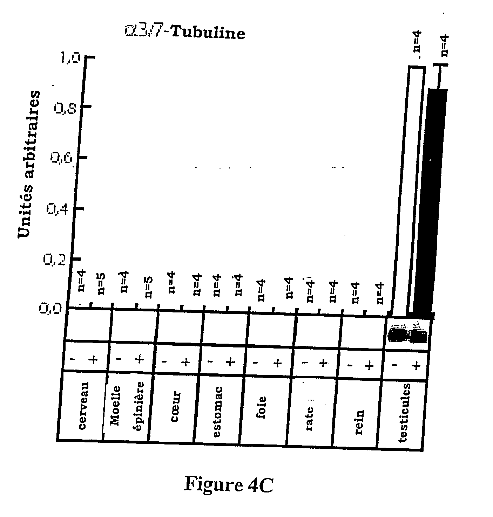

### 4 Accumulation d'ARNm de tubuline, profil d'expression et répartition des isotypes (Figure 4 et figure 5 et Tableau 2)

Le développement tout autant que la régénération axonale sont associés à un programme complexe d'expression des isotypes de tubuline régulés de façon temporelle et spatiale, reflétant souvent la densité des réseaux microtubulaires. Nous avons déterminé si les ARNm de tubuline variaient dans les échantillons des souris normales ou transgéniques d'âge croissant. En dépit d'une évaluation exhaustive de l'accumulation des ARNm, aucune différence évidente n'a été observée au cours du développement post natal des animaux.

L'analyse par Northern blot de cerveaux témoins et des échantillons de moelles épinières obtenus pendant le premier mois de croissance ex-utéro a révélé un net déclin pour les ARNm des deux isotypes de tubuline Mα1 et Mβ2. Ceci était suivi par le maintien d'un niveau bas tout au long de la maturité (Figures 4A, 5A).

Le profil d'expression des ARNm Mα2 et Mβ3 sont similaires entre les échantillons de cerveau de ceux de moelle épinière, tous deux étant présents à un niveau élevé à la naissance et restant élevés tout au long de la maturité ( figure 4B et 5B).

Pour chaque Northem blot, les signaux ont été normalisés par la GADPH qui n'est pas modifiée par l'agrégation des NF. (Robert, et al., 2001). Le signal obtenu à deux jours sert de référence et est fixé à 1 sauf pour la tubuline β5 2.8 kb(*), qui est comparée au signal de la tubuline 1.8 kb à 2 jours dans le cerveau contrôle. (ND: non déterminé à cause du signal trop faible).

Nous avons détecté un profil à deux phases pour le schéma d'expression des ARNm de tubuline Mβ4 dans les échantillons de cerveau et de moelle épinière (Figure 5C).

La séquence non traduite 3' de Mβ5 a reconnu deux transcrits de 1,8 et 2,8 kb (Figure 5D, 5E), qui ont eux aussi subi un fort déclin entre le deuxième jour post natal et la maturité à quatre semaines.

Enfin, le profil d'expression de la tubuline Mβ6 est assez similaire à ce qui a été observé pour la tubuline Mα1 (Figure 5F et 4A). Aucune indication de l'expression d'isoformes dérégulées n'a été détectée chez les souris transgéniques. Pour Mα3/7, normalement exprimé dans les testicules (Villasante et al., 1986) et Mβ1, normalement hématopoïetique-spécifique (Wang et al., 1986), aucun signal n'a été détecté dans les échantillons de cerveau et de moelle épinière, à aucune étape du développement. (Figure 4C).

Pour les isotypes de tubuline testés dans cette étude, aucune différence dans les profils d'expression des ARNm entre les échantillons transgéniques et contrôles n'a été observée, suggérant ainsi que des changements dans la densité de microtubules axonaux s'effectuent au niveau post-traductionnel.

Tableau 2A : La quantité d'ARNm de tubuline n'est pas modifiée chez les souris transgéniques.

| **A** | **CERVEAU** | | | | | | | |
|---|---|---|---|---|---|---|---|---|
| | **2 Jours** | | **4 Semaines** | | **Adultes** | | **18 Mois** | |
| | - | + | - | + | - | + | - | + |
| **α1 tubulin** | 1±0 | 0.82±0.05 | 0.11±0.03 | 0.12±0.4 | 0.1±0.01 | 0.12±0.04 | 0.8±0.01 | 0.08±0.01 |
| **α2 tubulin** | 1±0 | 1.06±0.3 | 0.96±0.18 | 0.78±0.07 | 0.68±0.07 | 0.94±0.26 | 1±0.17 | 0.93±0.07 |
| **β2 tubulin** | 1±0 | 0.88±0.9 | 0.42±0.0 | 0.41±0.16 | 0.47±0.18 | 0.33±0.1 | 0.6±0.22 | 0.5±0.03 |
| **β3 tubulin** | 1±0 | 1.09±0.11 | 1.08±0.32 | 1.1±0.11 | 0.89±0.07 | 1.01±0.13 | 0.9±0.09 | 0.9±0.03 |
| **β4 tubulin** | 1±0 | 1.19±0.08 | 2.84±0.08 | 3.07±0.05 | 1.39±0.1 | 1.89±0.33 | 1.61±0.24 | 1.55±0.34 |
| **β5 tubulin 1.8 kb** | 1±0 | 1.47±0.35 | | | | | | |
| **β5 tubulin 2.8 kb** | 11.35±2.93* | 11.04±2.34 | 0.66±0.11 | 0.92±0.31 | 0.61±0.21 | 0.59±0.48 | 0.79±0.54 | 0.75±0.54 |
| **β6 tubulin** | 1±0 | 1.08±0.05 | 0.18±0.04 | 0.17±0.07 | 0.13±0.02 | 0.14±0.02 | 0.17±0.02 | 0.14±0.02 |

| | **MOELLE EPINIERE** | | | | | | | |
|---|---|---|---|---|---|---|---|---|
| **α1 tubulin** | 0.6±0.24 | 0.51±0.19 | 0.19±0.09 | 0.1±0.05 | 0.08±0.04 | 0.1±0.05 | 0.6±0.05 | 0.7±0.04 |
| **α2 tubulin** | 0.95±0.31 | 0.94±0.06 | 0.89±0.16 | 0.83±0.15 | 1.15±0.06 | 0.82±0.12 | 0.86±0.24 | 0.79±0.31 |
| **β2 tubulin** | 3.15±0.1 | 2.19±0.2 | 0.55±0.14 | 0.4±0.11 | 0.31±0.01 | 0.28±0.03 | 0.15±0.03 | 0.14±0.02 |
| **β3 tubulin** | 1.1±0.18 | 0.93±0.0 | 0.95±0.0 | 0.89±0.05 | 1.02±0.2 | 0.94±0.24 | 0.96±0.2 | 1.13±0.18 |
| **β4 tubulin** | 1.33±0.62 | 1.2±0.37 | 4.73±0.98 | 5±0.04 | 3.7±0.44 | 2.65±0.04 | 2.77±0.07 | 3.12±0.25 |
| **β5 tubulin 1.8 kb** | 1.08±0.38 | 0.77±0.23 | | | | | | |
| **β5 tubulin 2.8 kb** | 9.64±4.13 | 8.3±6 | 1.12±0.13 | 0.75±0.62 | 0.35±0.2 | 0.87±0.62 | 0.85±0.68 | 0.42±0.24 |
| **β6 tubulin** | 0.52±0.1 | 0.47±0.08 | 0.21±0.08 | 0.18±0.05 | 0.15±0.06 | 0.15±0.06 | 0.13±0.05 | 0.14±0.08 |

### 5 Analyse de l'accumulation de microtubules et capacité de polymérisation (figure 6, tableau 2A & 2C).

Afin d'évaluer la quantité totale de tubuline accumulée, des homogénats bruts de cerveaux, moelles épinières et nerfs sciatiques ont été analysés par Western-Blot en utilisant des anticorps reconnaissant la tubuline α et β, les isoformes neuronales βIII, la forme polyglutamylée GT335, et la tubuline γ. Afin de comparer l'accumulation relative des isotypes, tous les échantillons ont été préparés sur le même gel et la même membrane de transfert a été analysée de façon séquentielle, avec tous les anticorps. Dans chaque cas, les anticorps de liaison ont été détectés en utilisant une réaction chemoluminescente et la quantité relative de tubuline entre-échantillon a été déterminée en mesurant les intensités des signaux sur le film. Les résultats des triplicatas d'expériences (3 dosages indépendants à partir des échantillons préparés à partir de deux souris adultes de chaque génotype) ont montré de façon convaincante que les échantillons transgéniques et contrôles contiennent les mêmes niveaux de chaque isoforme de tubuline recherchée. (Figure 6A, Tableau 2B).

### Tableau 2B : La quantité de protéines de tubuline n'est pas modifiée chez les souris transgéniques.

| **B** | **+/- ratio (%)** | | |
|---|---|---|---|
| | **CERVEAU** | **MOELLE EPINIERE** | **NERF SCIATIQUE** |
| **α-tub** | 97±3 | 105±8 | 97±5 |
| **β-tub** | 103±30 | 106±26 | 105±10 |
| **βIII-tub** | 99±7 | 119±21 | 91±9 |
| **GT335** | 104±2 | 126±33 | 94±12 |

Les signaux obtenus pour les souris contrôles ont été fixés à 100% et les ratios contrôles / transgéniques ont été calculés pour chaque expérience. Aucune différence dans la quantité de tubuline n'a été observée entre les souris contrôles et trangéniques.

Afin de déterminer si l'augmentation du nombre de microtubules axonaux peut être due à une augmentation de l'efficacité de la tubuline à polymériser, nous avons étudié la capacité relative de polymérisation de la tubuline à partir de cerveaux d'animaux transgéniques ou contrôles. Dans ce but, les protéines de microtubule ont été isolées par des cycles de polymérisation, à 37°C, et de dépolymérisation à 4°C (Weingarten et al., 1975). A chaque cycle, un aliquot est prélevé et analysé par Western-Blot, en utilisant le panel complet des anticorps anti-tubuline précédemment décrits, et par microscopie électronique. Dans un premier temps, le surnageant de l'extrait brut (S1) a été incubé à 37 °C et les microtubules assemblés ont été récupérés par sédimentation (P2). Les microtubules assemblés présents dans le deuxième culot ont été resuspendus puis dépolymérisés à 4°C avant d'être centrifugés pour obtenir de la tubuline libre (S3) et des « microtubules stables au froid » (P3). Aucune différence quantitative évidente n'a été observée entre les échantillons transgéniques et contrôles à n'importe quelle étape de cette procédure classique de purification des microtubules (Figure 6B).

Quand la même quantité de protéines a été déposée par puit dans le gel SDS PAGE, et que le ratio du signal pour l'immunoréactivité de la tubuline a été évalué (S2/P2 et S3/P3), les mêmes valeurs entre les échantillons transgéniques et de contrôles ont été obtenues (Tableau 2C).

**Tableau 2C : Les ratios entre la tubuline polymérisée et non polymérisée ne sont pas non plus modifiés chez les souris NFHLacZ dans le cerveau.**

| **C** | **CERVEAU** | | | |
|---|---|---|---|---|
| | **S2-/P2-** | **S2+/P2+** | **S3-/P3-** | **S3+/P3+** |
| **α-tub** | 0.68 ± 0.27 | 0.61 ± 0.20 | 1,22 ± 0.27 | 1,29 ± 0.19 |
| **β-tub** | 0.83 ± 0.16 | 0.79 ± 0.11 | 1,29 ± 0.27 | 1,12 ± 0.20 |
| **βIII-tub** | 0.38 ± 0.05 | 0.39 ± 0.02 | 1,60 ± 0.68 | 1,49 ± 0.32 |

Les mêmes ratios S2/P2 ont également été obtenus à partir des nerfs sciatiques des souris transgéniques ou contrôles (Tableau 2D).

Tableau 2D : Les ratios entre la tubuline polymérisée et non polymérisée ne sont pas modifiés non plus chez les souris NFHLacZ dans le nerf sciatique.

| **D** | **NERF SCIATIQUE** | |
|---|---|---|
| | **S2-/P2 -** | **S2+/P2+** |
| **α-tub** | 1.38 ± 0.78 | 1.39 ± 0.65 |
| **β-tub** | 1.33 ± 0.10 | 1.36 ± 0.47 |
| **βIII-tub** | 1.80 ± 0.58 | 2.06 ± 0.43 |

Tableaux 2C et D: Analyse par Western-Blot des différentes fractions d'une préparation de microtubules de cerveaux (C) ou de nerfs sciatiques (D) à l'aide de différents anticorps dirigés contre les tubulines α, β, βIII. Les signaux obtenus sont scannés et les ratios S2/P2 et S3/P3 ont été calculés. Aucune différence n'a été observée pour ces ratios entre les souris contrôles (-) et NFHLacZ (+).

Les ratios S3/P3 n'ont pas pu être évalués à cause de la faible quantité de protéines présente dans ces échantillons.

Afin de comparer également les propriétés de polymérisation de la tubuline, les extraits bruts (S1) des cerveaux transgéniques ou contrôles ont été incubés sous des conditions qui permettent la formation de microtubules (37°C ou en présence de Taxol (60µM)). Pour évaluer le potentiel relatif de dépolymérisation, de tels extraits bruts ont été incubés dans des conditions qui permettent le désassemblage des microtubules (4°C ou avec de la colchicine). Suivant de tels traitements, chaque préparation a été centrifugée et les microtubules ont été récupérés dans le culot et la tubuline non polymérisée a été récupérée dans le surnageant. A nouveau, aucune différence évidente dans l'intensité du signal pour les différents épitopes de tubuline dans ces échantillons de souris transgéniques ou contrôles n'a été observée (Figure 6C, D).

### 6 Analyses ultrastructurales et immunocytochimiques des agrégats de neurofilaments (Figure 7).

De précédentes études sur les souris transgéniques NFHLacZ ont démontré que les neurofilaments sont retenus en agrégats denses à l'intérieur du corps cellulaire (Eyer et Peterson, 1994). Afin de déterminer si de tels agrégats de neurofilaments exercent également une influence sur l'assemblage des microtubules *in vivo,* nous avons examiné dans les agrégats la présence de tubuline et de microtubules en utilisant à la fois l'immunohistochimie couplée à la microscopie confocale et la microscopie électronique. Bien que les profils de microtubules soient apparents dans le cytoplasme adjacent aux agrégats, aucun n'a été détecté, tant à l'intérieur qu'à la périphérie des agrégats (les profils complets de 50 agrégats ont été examinés à 50,000X) (Figure 7A). En contraste, dans les préparations immunocytochimiques évaluées par microscopie confocale, les épitopes de tubuline ont été clairement détectés à l'intérieur des agrégats (Figure 7B). Alors que les agrégats péricaryaux ont été fortement marqués avec des anticorps anti-neurofilament et anti-périphérine, le marquage le plus intense pour les épitopes de tubuline ont été observés, non à l'intérieur des agrégats, mais à la périphérie nucléaire et proche de la membrane cytoplasmique. Ainsi, alors que la tubuline est clairement détectable à l'intérieur des agrégats, elle peut être présente en de plus grande concentration dans d'autres compartiments sub-cellulaires. En contraste avec la distribution hétérogène des épitopes de tubuline observée à l'intérieur des neurones NFHLacZ, un marquage homogène péricarial a été observé dans des neurones normaux, chevauchant les signaux dérivés des anticorps monoclonaux anti βIII tubuline et des anticorps polyclonaux anti-NFH ou anti-périphérine (Figure 7B). Des distributions similaires ont également été observées dans des neurones transgéniques et normaux pour la tubuline α-, β-, et γ (non montré).

### ► Exemple 2. Les peptides NFL-TBS2 bloquent la division cellulaire

Les peptides TBS modifient l'assemblage in situ des microtubules, la mitose et la prolifération de cellules.

Les peptides de NFL-TBS2 démontrent un effet puissant sur la polymérisation in vitro de la tubuline. Pour déterminer s'ils pourraient également modifier l'assemblage in-situ des microtubules, des cellules de T98G ont été incubées avec 10µm de peptides SEQ ID N°6 biotinylés. Après une incubation de 6 heures, plus de 75 % des cellules contiennent le peptide (figure 8 A, A', A") et sont caractérisées par une forme sphérique peu commune. Les cellules avec un signal mesurant la présence des peptides faible ou absent ont maintenu un cytosquelette normal de microtubule, mais celles contenant le peptide SEQ ID N°6 ont montré un réseau désuni de microtubules avec la tubuline et les peptides agrégés dans une masse amorphe.

Les microtubules constituent un composant important du fuseau achromatique. Pour déterminer si la présence de peptides SEQ ID N°6 pourrait affecter la division de cellules, les inventeurs ont l'habitude d'effectuer une analyse FACS (de l'anglais Fluorescence Activated Cell Sorter ) pour comparer la répartition des cycles de cellules de contrôle et des cellules T98G traitées avec 10µm de peptide SEQ ID N N°6.

Après une culture de 48 heures, 39% des cellules dans les cultures non traitées avec le peptide SEQ ID N°6 étaient en phase G1 de mitose. En revanche, 61% des cellules dans les cultures contenant le peptide SEQ ID N°6 étaient en phase G1 de mitose, suggérant ainsi l'arrêt de la mitose (figure 8 B, B').

L'effet de l'exposition du peptide SEQ ID N°6 sur la prolifération des cellules a été confirmé en utilisant une analyse MTS (décrit dans Cory A.H. et al. 1991, Cancer Commun. 3, 207-212, incorporé ici par référence) où les inventeurs ont observé moins de cellules dans les cultures traitées par le peptide SEQ ID N°6 (Figure 8C). Dans d'autres expériences, des milieux contenant le peptide SEQ ID N°6 ont été remplacés à 24 heures (pour éventuellement palier à la dégradation du peptide lors de l'incubation), et l'inhibition de l'augmentation du nombre de cellules aux étapes suivant la culture a aussi été clairement observée (données non montrées).

Ainsi, après cette durée de culture, ces résultats suggèrent que le peptide SEQ ID N°6 est peu (ou pas) dégradé ou éliminé.

Des résultats semblables sont observés en utilisant d'autres lignées de cellules, par exemple MCF7, LS 187, Cos ou cellules de NGP.

### ► Exemple 3. Des peptides d'autres filaments intermédiaires se lient à la tubuline, altèrent la polymérisation des microtubules et bloquent la division cellulaire

### Liaison de peptides issus d'autres filaments intermédiaires à la tubuline

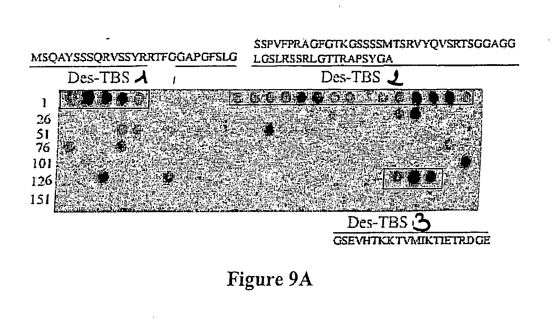

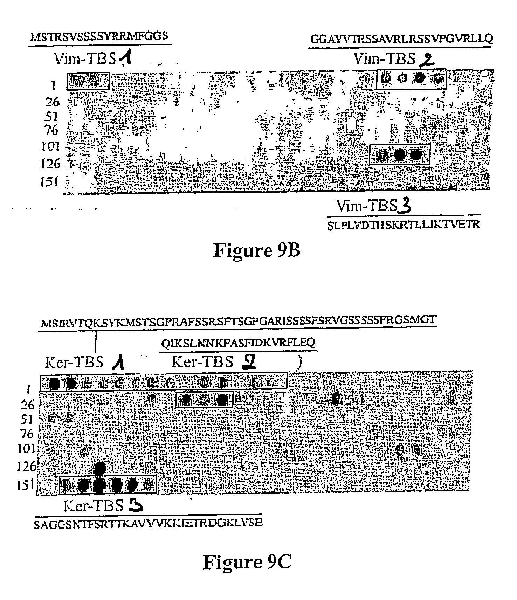

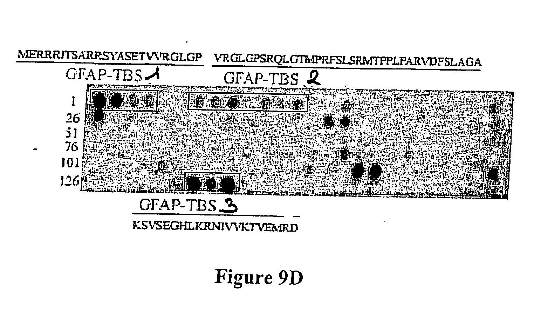

Afin de tester directement si d'autres filaments intermédiaires peuvent lier la tubuline, des puces peptidiques correspondant aux séquences de la desmine, de la vimentine, de la cytokératine et de la GFAP ont été préparées et évaluées comme décrit dans la partie Matériel et Méthode (Puce peptidique sur membrane et peptides synthétiques) de l'Exemple 1. Chacun de ses filaments intermédiaires ont plusieurs sites de liaison à la tubuline :
- 3 peptides ont été identifiés pour la protéine desmine (Figure 9A) : Des-TBS 1 (SEQ ID NO :17), Des-TBS2 (SEQ ID NO :18), et Des-TBS3 (SEQ ID NO :19).
- 3 peptides ont été identifiés pour la protéine vimentine (Figure 9B) : Vim-TBS 1 (SEQ ID NO :20), Vim-TBS2 (SEQ ID NO :21), et Vim-TBS3 (SEQ ID NO :22)
- 3 peptides ont été identifiés pour la protéine cytokératine (Figure 9C) : Ker-TBS 1 (SEQ ID NO :23), Ker-TBS2 (SEQ ID NO :24), et Ker-TBS3 (SEQ ID NO :25)
- 3 peptides ont été identifiés pour la protéine GFAP (Figure 9D) : GFAP-TBS1 (SEQ ID NO :26), GFAP-TBS2 (SEQ ID NO :27), et GFAP-TBS3 (SEQ ID NO :28).

### Effet des peptides issus d'autres filaments intermédiaires sur la polymérisation des microtubules

Comme précédemment pour les peptides NF-TBS, des études de l'influence des peptides de la vimentine, de la cytokératine et de la GFAP identifiés par puce peptidique sur l'assemblage des microtubules in vitro ont été réalisées par la même technique que décrit précédemment dans la partie Matériel et Méthode (Analyse par spectrophotométrie de la polymérisation des microtubules) de l'Exemple 1.

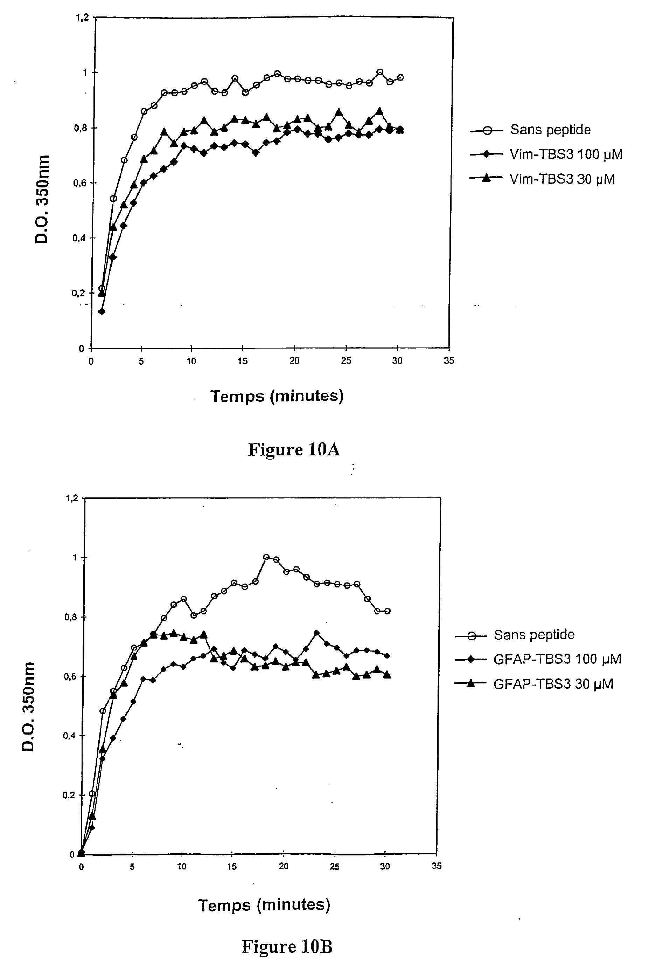

Les résultats obtenus pour les peptides Vim-TBS3 (SEQ ID NO : 22), GFAP-TBS3 (SEQ ID NO : 28), Ker-TBS1 (SEQ ID NO : 23), Vim-TBS2 (SEQ ID NO : 21) et GFAP-TBS1 (SEQ ID NO : 26) sont représentés sur les Figure 10A à 10E.

Les résultats montrent que les peptides Vim-TBS3 (SEQ ID NO : 22) et GFAP-TBS3 (SEQ ID NO : 28), de même que le peptide NFL-TBS2 (SEQ ID NO : 6), sont des inhibiteurs de la polymérisation des microtubules (Figure 10A et B).

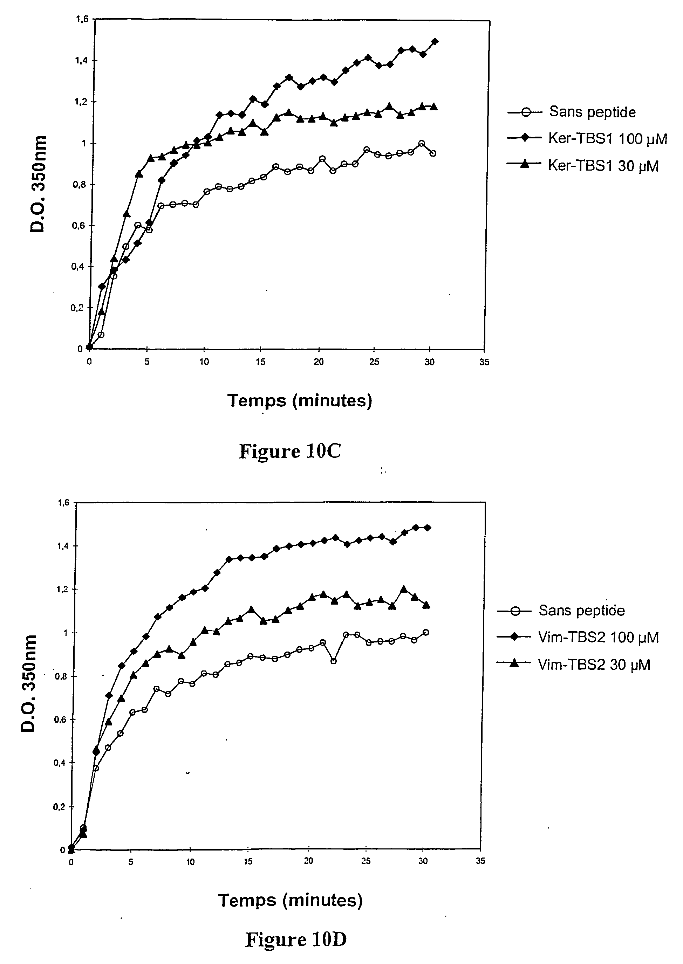

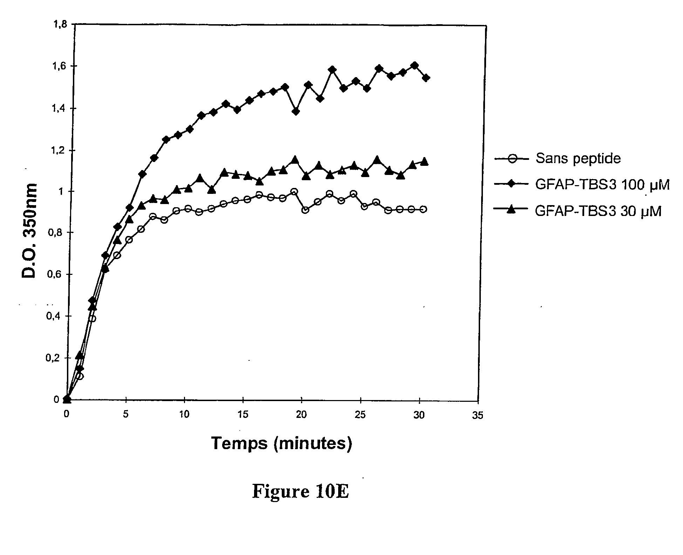

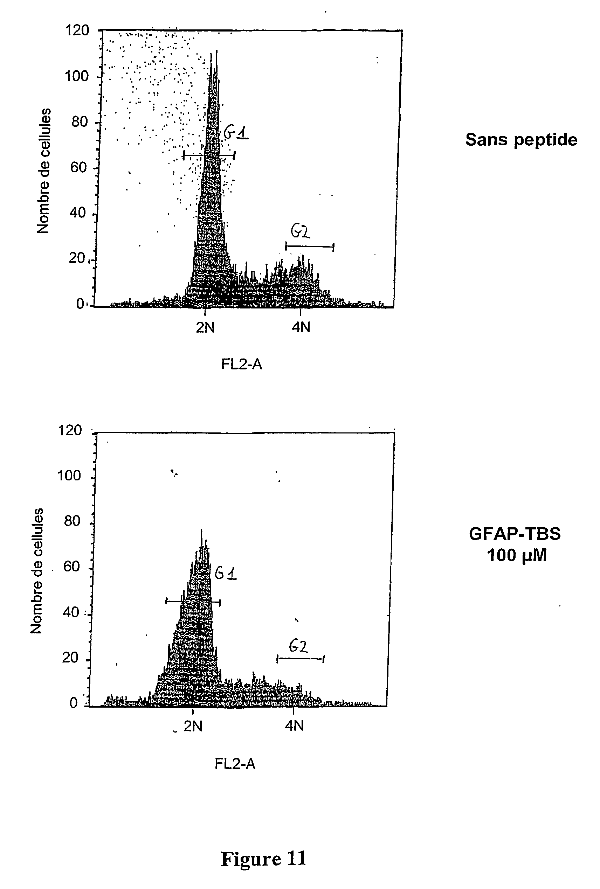

Au contraire, les peptides Ker-TBS1 (SEQ ID NO : 23), Vim-TBS2 (SEQ ID NO : 21) et GFAP-TBS1 (SEQ ID NO : 26) activent la polymérisation des microtubules (Figure 10C, D et E).

### Effet du peptide GFAP-TBS1 (SEQ ID NO : 26) sur la division cellulaire

Afin de déterminer l'impact d'un peptide activateur de la polymérisation des microtubules sur la division cellulaire, une analyse FACS a été réalisée pour comparer la répartition des cycles de cellules T98G non traitées (sans peptide) ou traitées avec 100 µm de peptide GFAP-TBS1 (SEQ ID NO : 26), par le même protocole que celui décrit dans l'Exemple 2.

Comme dans l'Exemple 2 avec le peptide NFL-TBS2 (SEQ ID NO : 6), on observe en présence du peptide GFAP-TBS1 (SEQ ID NO : 26) une diminution du nombre de cellules en phase G2 et une augmentation du nombre de cellules en phase G1 ou sub-G1, indiquant que la présence du peptide GFAP-TBS1 (SEQ ID NO : 26) inhibe la division cellulaire.

Ainsi, ces résultats suggèrent fortement que les peptides issus de filaments intermédiaires se liant à la tubuline sont capables d'altérer la polymérisation des microtubules (inhibition ou activation), et de ce fait, par l'un ou l'autre mécanisme, bloquent la division cellulaire.

En conclusion, les relations définies ici entre des fragments de filaments intermédiaires et la tubuline peuvent exemplifier un mécanisme largement exploité de régulation des microtubules.

Les motifs correspondant à NF-TBS ont été particulièrement étudiés ici et plusieurs de ces derniers sont mutés dans diverses maladies humaines comme la Sclérose Latérale Amyotrophique, ou des maladies de la peau, et dont les différentes mutations sont listées sur le site www.interfil.org. De plus, plusieurs de tels résidus de liaison TBS sont sujets à la phosphorylation dépendante des cycles des cellules, ce qui suggère leur implication possible dans les équilibres liaison/dégagement de la tubuline (Chou et al. 1996, Goto et al. 1998, Ku et al. 1998, Toivola et al. 2002, Chou et al. 2003).

Les résultats montrent également que des fragments d'autres filaments intermédiaires (desmine, vimentine, cytokératine et GFAP) possèdent également des fragments capables de se lier à le tubuline, d'altérer la polymérisation des microtubules (inhibition ou activation) et de bloquer la division cellulaire, mettant ainsi en évidence l'existence d'un mécanisme commun et important du contrôle de la division cellulaire par altération de la polymérisation des microtubules.

### References

Carden, M.J., Trojanowski, J.Q., Schlaepfer, W.W., and Lee, V.M.Y. (1987). Two stage expression of neurofilament polypeptides during rat neurogenesis with early establishment of adult phosphorylation patterns. J. Neurosci. 7, 3489-3504.
Cassimeris, L. (2002). The oncoprotein 18/stathmin family of microtubule destabilizers. Curr. Opin. Cell Biol. 14, 18-24.
Chomczynski, P., and Sacchi, N. (1987). Single-step method of RNA isolation by acid guanidinium thiocyanate-phenol-chloroform extraction. Anal. Biochem. 162, 156-159.
Chou. Y.H., Opal, P., Quinlan, R.A., and Goldman, R.D. (1996). The relative roles of specific N- and C-terminal phosphorylation sites in the disassembly of intermediate filament in mitotic BHK-21 cells. J. Cell Sci. 109, 817-826.
Chou. Y.H., Khuon, S., Herrmann, H., and Goldman, R.D. (2003). Nestin promotes the phosphorylation-dependent disassemble of vimentin intermediate filaments during mitosis. Mol. Biol. Cell.14, 1468-1478.
Cory AH, Owen TC, Barltrop JA, Cory JG. (1991). Use of an aqueous soluble tetrazolium/formazan assay for cell growth assays in culture., Cancer Commun. 3, 207-212.
De Waegh., S.M., Lee, V.M., and Brady, S.T. (1992). Local modulation of neurofilament phosphorylation, axonal caliber, and slow axonal transport by myelinating Schwann cells. Cell 68, 451-463.
Eyer, J., and Leterrier, J.F. (1988). Influence of the phosphorylation state of neurofilament proteins on the in vitro interactions between purified filaments. Biochem. J. 252, 655-660.
Eyer, J., and Peterson, A.C. (1994). Neurofilament-deficient axons and perikaryal aggregates in viable transgenic mice expressing a neurofilament-beta-galactosidase fusion protein. Neuron 12, 389-405.
Fort, P., Marty, L., Piechaczyk, M., el Sabrouty, S., Dani, C., Jeanteur, P., and Blanchard, J.M. (1985). Various rat adult tissues express only one major mRNA species from the glyceraldehyde-3-phosphate-dehydrogenase multigenic family. Nucleic Acids Res. 13, 1431-1442.
Frank, R. (1992) Spot-Synthesis: An easy technique for the positionally addressable, parallel chemical synthesis on a membrane support. Tetrahedron 48, 9217-9232.
Frank, R. and Overwin, H. (1996) SPOT-Synthesis: Epitope analysis with arrays of synthetic peptides prepared on cellulose membranes. In: Methods in Molecular Biology, Vol. 66: Epitope Mapping Protocols (G. E. Morris, Ed.), The Humana Press Inc., Totowa, USA, 149-169.
Goto, H., Kosako, H., Tanabe, K., Yanagida; M., Sakurai, M., Amano, M., Kaibuchi, K., Inagaki, M. (1998) Phosphorylation of vimentin by Rho-associated kinase at a unique amino-terminal site that is specifically phosphorylated during cytokinesis. J. Biol. Chem. 273,11728-11736.
Guillaud, L., Bosc, C., Fourest-Lieuvin, A., Denarier, E., Pirollet, F., Lafanechere, L., and Job, D. (1998). STOP proteins are responsible for the high degree of microtubule stabilization observed in neuronal cells. J. Cell Biol. 142, 167-179.
Hirokawa, N. (1982). Cross-linker system between neurofilaments, microtubules, and membranous organelles in frog axons revealed by the quick-freeze, deep-etching method. J. Cell Biol. 94, 129-142.
Hisanaga, S. and Hirokawa, N. (1990). Dephosphorylation-induced interactions of neurofilaments with microtubules. J. Biol. Chem. 265, 21852-21858.
Hoffman, P.N., and Cleveland, D.W., (1988). Neurofilament and tubulin expression recapitulates the developmental program during axonal regeneration: induction of a specific beta-tubulin isotype. Proc. Natl. Acad. Sci USA. 85, 4530-4533.
Hoffman, P.N., Cleveland, D.W., Griffin, J.W., Landes, P.W., Cowan, N.J., and Price, D.L. (1987). Neurofilament gene expression: a major déterminant of axonal caliber. Proc. Natl. Acad. Sci.-USA. 84, 3472-3476.
Julien, J.P., and Mushynski, W.E. (1982) Multiple phosphorylation sites in mammalian neurofilament polypeptides. J. Biol. Chem. 257, 10467-10470.
Ku, N.O., Liao, J., and Omary, M.B. (1998) Phosphorylation of human keratin 18 serine 33 regulates binding to 14-3-3 proteins. EMBO J. 17, 1892-1906.
Laemmli, U.K. (1970). Cleavage of structural proteins during the assembly of the head ofbacteriophage T4. Nature 227, 680-685.
Lee, V.M.Y., Carden, M.J., Schlaepfer, W.W., Trojanowski, J.Q., Otvos, L., Hollosi, M., Dietzchold, B., Lazzarini, R.A. (1988). Identification of the major multiphosphorylation sites in mammalian neurofilaments. Proc. Natl. Acad. Sci.-USA 85, 1998-2002.
Mandelkow, E., and Mandelkow, E.M. (1995). Microtubules and microtubule-associated proteins. Curr. Opin. Cell Biol 7, 72-81.
Ohara, O., Gahara, Y., Miyake, T., Teraoka, H., and Kitamura, T. (1993). Neurofilament deficiency in quail caused by nonsense mutation in neurofilament-L gene. J. Cell Biol.121, 387-395.
Robert, P., Peterson, A., and Eyer, J. (2001). Neurofilament cytoskeleton does not modify accumulation of trophic factors mRNA. J. Neurosci. Res. 64, 487-492.
Toivola, D.M., Zhou, Q., English, L.S., and Omary, M.B. (2002) Type II keratins are phosphorylated on a unique motif during stress and mitosis in tissues and cultured cells. Mol. Biol. Cell 13, 1857-1870.
Weingarten, M.D., Lockwood, A.H., Hwo S.-Y., and Kirschner, M.W. (1975). A protein factor essential for microtubule assembly. Proc. Natl. Acad. Sci. USA. 72, 1858-1862.
Zhu, Q., Couillard-Despres, S., and Julien, J.P. (1997). Delayed maturation of regenerating myelinated axons in mice lacking neurofilaments. Exp. Neurol. 148, 299-316.
Zhu, Q., Lindenbaum, M., Levavasseur, F., Jacomy, H., and Julien, J.P. (1998). Disruption of the NFH gene increases axonal microtubule content and velocity of neurofilament transport: relief of axonopathy resulting from the toxin beta,beta'-iminodipropionitrile. J. Cell Biol. 143, 183-193.

## Revendications

1. Peptide isolé **caractérisé en ce qu'**il consiste en un peptide présentant au moins 80 % de préférence 85 %, 90 %, 95 %, 98 % et 100% d'identité après alignement optimal avec une séquence choisie parmi SEQ ID N°5, SEQ ID N°6, SEQ ID N°7 SEQ ID N°8, SEQ ID N°17, SEQ ID N°18, SEQ ID N°19, SEQ ID N°20, SEQ ID N°21, SEQ ID N°22, SEQ ID N°23, SEQ ID N°24, SEQ ID N°25, SEQ ID N°26, SEQ ID N°27 ou SEQ ID N°28 ou l'un de ses fragments biologiques artificiel d'au moins 5 aminoacides, capable d'altérer la polymérisation de la tubuline.

2. Peptide selon la revendication 1, de séquence choisie parmi SEQ ID N°5, SEQ ID N°6, SEQ ID N°7, SEQ ID N°8, SEQ ID N°17, SEQ ID N°18, SEQ ID N°19, SEQ ID N°20, SEQ ID N°21, SEQ ID N°22, SEQ ID N°23, SEQ ID N°24, SEQ ID N°25, SEQ ID N°26, SEQ ID N°27 ou SEQ ID N°28.

3. Peptide selon l'une quelconque des revendications 1 et 2 **caractérisé en ce qu'**il est obtenu par voie recombinante ou par synthèse chimique.

4. Acide nucléique isolé codant pour un peptide selon l'une quelconque des revendications 1 à 3.

5. Acide nucléique selon la revendication 4 présentant au moins *80* %, de préférence 85%, *90* %, *95* %, *98* % et 100% d'identité après alignement optimal avec une séquence choisie parmi SEQ ID N°1, SEQ ID N°2, SEQ ID N°3, SEQ ID N°4, SEQ ID N°29, SEQ ID N°30, SEQ ID N°31, SEQ ID N°32, SEQ ID N°33, SEQ ID N°34, SEQ ID N°35, SEQ ID N°36, SEQ ID N°37, SEQ ID N°38, SEQ ID N°39 ou SEQ ID N°40.

6. Vecteur **caractérisé en ce qu'**il comprend un acide nucléique selon l'une quelconque des revendications 4 et 5.

7. Cellule hôte **caractérisée en ce qu'**elle est transformée par un acide nucléique selon l'une quelconque des revendications 4 et 5, ou un vecteur selon la revendication 6.

8. Procédé de production de peptide recombinant selon l'une quelconque des revendications 1 à 3, **caractérisé en ce qu'**il comprend les étapes suivantes :
a) culture d'une cellule selon la revendication 7 dans un milieu de culture et des conditions de culture appropriées,
b) récupération dudit peptide à partir des cellules ou du milieu de culture obtenu à l'étape a).

9. Composition pharmaceutique comprenant un peptide selon l'une quelconque des revendications 1 à 3 et/ou obtenu par un procédé selon la revendication 8, avec un véhicule pharmaceutiquement acceptable.

10. Peptide selon l'une quelconque des revendications 1 à 3 et/ou obtenu par un procédé selon la revendication 8 pour une utilisation pour inhiber la prolifération cellulaire et/ou la mobilité cellulaire, afin de prévenir ou traiter une maladie choisie parmi les cancers, les infections bactériennes, les maladies impliquant une prolifération de certaines plantes comme les algues ou les lichens, ou le psoriasis.

11. Peptide selon la revendication 10 pour une utilisation pour inhiber l'angiogénèse au cours de la croissance tumorale.

12. Peptide selon la revendication 10 pour une utilisation pour inhiber la multiplication bactérienne.

13. Peptide selon l'une quelconque des revendications 1 à 3 et/ou obtenu par un procédé selon la revendication 8 pour une utilisation *in vitro* comme marqueur de la tubuline.

14. Peptide selon l'une quelconque des revendications 1 à 3 et/ou obtenu par un procédé selon la revendication 8, pour une utilisation en tant que médicament.

15. Peptide selon la revendication 14, **caractérisé en ce que** le médicament est destiné à prévenir ou traiter des maladies impliquant une prolifération cellulaire et/ou une motilité cellulaire à savoir les infections bactériennes, les maladies impliquant une prolifération de certaines plantes comme les algues ou les lichens, ou le psoriasis.

16. Peptide selon la revendication 14, **caractérisé en ce que** le médicament est destiné à prévenir ou traiter des cancers.

17. Peptide selon la revendication 16, **caractérisé en ce que** le cancer est un cancer du système nerveux.

18. Utilisation d'un peptide selon l'une quelconque des revendications 1 à 3 et/ou obtenu par un procédé selon la revendication 8 pour la mise en évidence et/ou pour tester *in vitro* des produits susceptibles de bloquer l'interaction entre la tubuline et ledit peptide.

19. Utilisation d'un fragment de filament intermédiaire capable d'altérer la polymérisation de la tubuline choisi parmi la cytokératine endoA présentant au moins 80%, de préférence 85%, 90%, 98% et 100% d'identité après alignement optimal avec la séquence SEQ ID N°9, la kératine 8 présentant au moins 80%, de préférence 85%, 90%, 98% et 100% d'identité après alignement optimal avec la séquence SEQ ID N°10, la périphérine présentant au moins 80%, de préférence 85%, 90%, 98% et 100% d'identité après alignement optimal avec la séquence SEQ ID N°11, l'internexine présentant au moins 80%, de préférence 85%, 90%, 98% et 100% d'identité après alignement optimal avec la séquence SEQ ID N°12, la GFAP présentant au moins 80%, de préférence 85%, 90%, 98% et 100% d'identité après alignement optimal avec la séquence SEQ ID N°13 ou la vimentine présentant au moins 80%, de préférence 85%, 90%, 98% et 100% d'identité après alignement optimal avec la séquence SEQ ID N°14 pour la fabrication d'un médicament destiné à prévenir ou à traiter des maladies impliquant la prolifération cellulaire et/ou la mobilité cellulaire, choisies parmi les cancers, les infections bactériennes, les maladies impliquant une prolifération de certaines plantes comme les algues ou les lichens, ou le psoriasis.

20. Utilisation *in vitro* d'un fragment de filament intermédiaire capable d'altérer la polymérisation de la tubuline choisi parmi la cytokératine endoA présentant au moins 80%, de préférence 85%, 90%, 98% et 100% d'identité après alignement optimal avec la séquence SEQ ID N°9, la kératine 8 présentant au moins 80%, de préférence 85%, 90%, 98% et 100% d'identité après alignement optimal avec la séquence SEQ ID N°10, la périphérine présentant au moins 80%, de préférence 85%, 90%, 98% et 100% d'identité après alignement optimal avec la séquence SEQ ID N°11, l'internexine présentant au moins 80%, de préférence 85%, 90%, 98% et 100% d'identité après alignement optimal avec la séquence SEQ ID N°12, la GFAP présentant au moins 80%, de préférence 85%, 90%, 98% et 100% d'identité après alignement optimal avec la séquence SEQ ID N°13 ou la vimentine présentant au moins 80%, de préférence 85%, 90%, 98% et 100% d'identité après alignement optimal avec la séquence SEQ ID N°14 comme marqueur de la tubuline.

21. Fragment de filament intermédiaire capable d'altérer la polymérisation de la tubuline choisi parmi la cytokératine endoA présentant au moins 80%, de préférence 85%, 90%, 98% et 100% d'identité après alignement optimal avec la séquence SEQ ID N°9, la kératine 8 présentant au moins 80%, de préférence 85%, 90%, 98% et 100% d'identité après alignement optimal avec la séquence SEQ ID N°10, la périphérine présentant au moins 80%, de préférence 85%, 90%, 98% et 100% d'identité après alignement optimal avec la séquence SEQ ID N°11, l'internexine présentant au moins 80%, de préférence 85%, 90%, 98% et 100% d'identité après alignement optimal avec la séquence SEQ ID N°12, la GFAP présentant au moins 80%, de préférence 85%, 90%, 98% et 100% d'identité après alignement optimal avec la séquence SEQ ID N°13 ou la vimentine présentant au moins 80%, de préférence 85%, 90%, 98% et 100% d'identité après alignement optimal avec la séquence SEQ ID N°14 pour une utilisation en tant que médicament.

22. Utilisation d'un fragment de filament intermédiaire capable d'altérer la polymérisation de la tubuline choisi parmi la cytokératine endoA présentant au moins 80%, de préférence 85%, 90%, 98% et 100% d'identité après alignement optimal avec la séquence SEQ ID N°9, la kératine 8 présentant au moins 80%, de préférence 85%, 90%, 98% et 100% d'identité après alignement optimal avec la séquence SEQ ID N°10, la périphérine présentant au moins 80%, de préférence 85%, 90%, 98% et 100% d'identité après alignement optimal avec la séquence SEQ ID N°11, l'internexine présentant au moins 80%, de préférence 85%, 90%, 98% et 100% d'identité après alignement optimal avec la séquence SEQ ID N°12, la GFAP présentant au moins 80%, de préférence 85%, 90%, 98% et 100% d'identité après alignement optimal avec la séquence SEQ ID N°13 ou la vimentine présentant au moins 80%, de préférence 85%, 90%, 98% et 100% d'identité après alignement optimal avec la séquence SEQ ID N°14 pour la mise en évidence et/ou pour tester *in vitro* des produits susceptibles de bloquer l'interaction entre la tubuline et ledit fragment de filament intermédiaire.

## Claims

1. Isolated peptide, **characterized in that** it consists of a peptide having at least 80% and preferably 85%, 90%, 95%, 98% and 100% of identity after optimum alignment with a sequence selected from SEQ ID No. 5, SEQ ID No. 6, SEQ ID No. 7, SEQ ID No. 8, SEQ ID No. 17, SEQ ID No. 18, SEQ ID No. 19, SEQ ID No. 20, SEQ ID No. 21, SEQ ID No. 22, SEQ ID No. 23, SEQ ID No. 24, SEQ ID No. 25, SEQ ID No. 26, SEQ ID No. 27 or SEQ ID No. 28 or one of its artificial biological fragments of at least 5 amino acids, capable of altering tubulin polymerization.

2. Peptide according to claim 1, of sequence selected from SEQ ID No. 5, SEQ ID No. 6, SEQ ID No. 7, SEQ ID No. 8, SEQ ID No. 17, SEQ ID No. 18, SEQ ID No. 19, SEQ ID No. 20, SEQ ID No. 21, SEQ ID No. 22, SEQ ID No. 23, SEQ ID No. 24, SEQ ID No. 25, SEQ ID No. 26, SEQ ID No. 27 or SEQ ID No. 28.

3. Peptide according to either one of claims 1 and 2, **characterized in that** it is obtained by a recombinant technique or by chemical synthesis.

4. Isolated nucleic acid coding for a peptide according to any one of claims 1 to 3.

5. Nucleic acid according to claim 4, having at least 80% and preferably 85%, 90%, 95%, 98% and 100% of identity after optimum alignment with a sequence selected from SEQ ID No. 1, SEQ ID No. 2, SEQ ID No. 3, SEQ ID No. 4, SEQ ID No. 29, SEQ ID No. 30, SEQ ID No. 31, SEQ ID No. 32, SEQ ID No. 33, SEQ ID No. 34, SEQ ID No. 35, SEQ ID No. 36, SEQ ID No. 37, SEQ ID No. 38, SEQ ID No. 39 or SEQ ID No. 40.

6. Vector **characterized in that** it comprises a nucleic acid according to either one of claims 4 and 5.

7. Host cell **characterized in that** it is transformed by a nucleic acid according to either one of claims 4 and 5, or a vector according to claim 6.

8. Method of production of recombinant peptide according to any one of claims 1 to 3, **characterized in that** it comprises the following stages:
a) culture of a cell according to claim 7 in a culture medium and suitable culture conditions,
b) recovery of said peptide from the cells or from the culture medium obtained in stage a).

9. Pharmaceutical composition comprising a peptide according to any one of claims 1 to 3 and/or obtained by a method according to claim 8, with a pharmaceutically acceptable vehicle.

10. Peptide according to any one of claims 1 to 3 and/or obtained by a method according to claim 8 for use in inhibiting cell proliferation and/or cell mobility, so as to prevent or treat a disease selected from cancers, bacterial infections, diseases involving proliferation of certain plants such as algae or lichens, or psoriasis.

11. Peptide according to claim 10 for use in inhibiting angiogenesis during tumor growth.

12. Peptide according to claim 10 for use in inhibiting bacterial multiplication.

13. Peptide according to any one of claims 1 to 3 and/or obtained by the method according to claim 8 for *in vitro* use as a tubulin marker.

14. Peptide according to any one of claims 1 to 3 and/or obtained by the method according to claim 8, for use as a medicinal product.

15. Peptide according to claim 14, **characterized in that** the medicinal product is intended for the prevention or treatment of diseases involving cell proliferation and/or cell motility, namely bacterial infections, diseases involving proliferation of certain plants such as algae or lichens, or psoriasis.

16. Peptide according to claim 14, **characterized in that** the medicinal product is intended for the prevention or treatment of cancers.

17. Peptide according to claim 16, **characterized in that** the cancer is a cancer of the nervous system.

18. Use of a peptide according to any one of claims 1 to 3 and/or obtained by the method according to claim 8 for the detection and/or *in vitro* testing of products that are able to block interaction between tubulin and said peptide.

19. Use of a fragment of intermediate filament capable of altering tubulin polymerization selected from the cytokeratin endoA having at least 80% and preferably 85%, 90%, 98% and 100% of identity after optimum alignment with the sequence SEQ ID No. 9, keratin 8 having at least 80% and preferably 85%, 90%, 98% and 100% of identity after optimum alignment with the sequence SEQ ID No. 10, peripherin having at least 80% and preferably 85%, 90%, 98% and 100% of identity after optimum alignment with the sequence SEQ ID No. 11, internexin having at least 80% and preferably 85%, 90%, 98% and 100% of identity after optimum alignment with the sequence SEQ ID No. 12, GFAP having at least 80% and preferably 85%, 90%, 98% and 100% of identity after optimum alignment with the sequence SEQ ID No. 13 or vimentin having at least 80% and preferably 85%, 90%, 98% and 100% of identity after optimum alignment with the sequence SEQ ID No. 14, for the production of a medicinal product intended for the prevention or treatment of diseases involving cell proliferation and/or cell motility, selected from cancers, bacterial infections, diseases involving proliferation of certain plants such as algae or lichens, or psoriasis.

20. *In vitro* use of a fragment of intermediate filament capable of altering tubulin polymerization selected from the cytokeratin endoA having at least 80% and preferably 85%, 90%, 98% and 100% of identity after optimum alignment with the sequence SEQ ID No. 9, keratin 8 having at least 80% and preferably 85%, 90%, 98% and 100% of identity after optimum alignment with the sequence SEQ ID No. 10, peripherin having at least 80% and preferably 85%, 90%, 98% and 100% of identity after optimum alignment with the sequence SEQ ID No. 11, internexin having at least 80% and preferably 85%, 90%, 98% and 100% of identity after optimum alignment with the sequence SEQ ID No. 12, GFAP having at least 80% and preferably 85%, 90%, 98% and 100% of identity after optimum alignment with the sequence SEQ ID No. 13 or vimentin having at least 80% and preferably 85%, 90%, 98% and 100% of identity after optimum alignment with the sequence SEQ ID No. 14, as a tubulin marker.

21. Fragment of intermediate filament capable of altering tubulin polymerization selected from the cytokeratin endoA having at least 80% and preferably 85%, 90%, 98% and 100% of identity after optimum alignment with the sequence SEQ ID No. 9, keratin 8 having at least 80% and preferably 85%, 90%, 98% and 100% of identity after optimum alignment with the sequence SEQ ID No. 10, peripherin having at least 80% and preferably 85%, 90%, 98% and 100% of identity after optimum alignment with the sequence SEQ ID No. 11, internexin having at least 80% and preferably 85%, 90%, 98% and 100% of identity after optimum alignment with the sequence SEQ ID No. 12, GFAP having at least 80% and preferably 85%, 90%, 98% and 100% of identity after optimum alignment with the sequence SEQ ID No. 13 or vimentin having at least 80% and preferably 85%, 90%, 98% and 100% of identity after optimum alignment with the sequence SEQ ID No. 14, for use as a medicinal product.

22. Use of a fragment of intermediate filament capable of altering tubulin polymerization selected from the cytokeratin endoA having at least 80% and preferably 85%, 90%, 98% and 100% of identity after optimum alignment with the sequence SEQ ID No. 9, keratin 8 having at least 80% and preferably 85%, 90%, 98% and 100% of identity after optimum alignment with the sequence SEQ ID No. 10, peripherin having at least 80% and preferably 85%, 90%, 98% and 100% of identity after optimum alignment with the sequence SEQ ID No. 11, internexin having at least 80% and preferably 85%, 90%, 98% and 100% of identity after optimum alignment with the sequence SEQ ID No. 12, GFAP having at least 80% and preferably 85%, 90%, 98% and 100% of identity after optimum alignment with the sequence SEQ ID No. 13 or vimentin having at least 80% and preferably 85%, 90%, 98% and 100% of identity after optimum alignment with the sequence SEQ ID No. 14, for detecting and/or *in vitro* testing products that are able to block interaction between tubulin and said fragment of intermediate filament.

## Patentansprüche

1. Isoliertes Peptid, **dadurch gekennzeichnet, dass** es ein Peptid ist, das nach optimalem Sequenzalignment mindestens 80 %, vorzugsweise 85 %, 90 %, 95 %, 98 % und 100 % Identität mit einer Sequenz aufweist, die ausgewählt ist aus SEQ ID Nr. 5, SEQ ID Nr. 6, SEQ ID Nr. 7 SEQ ID Nr. 8, SEQ ID Nr. 17, SEQ ID Nr. 18, SEQ ID Nr. 19, SEQ ID Nr. 20, SEQ ID Nr. 21, SEQ ID Nr. 22, SEQ ID Nr. 23, SEQ ID Nr. 24, SEQ ID Nr. 25, SEQ ID Nr. 26, SEQ ID Nr. 27 oder SEQ ID Nr. 28, oder von einem seiner künstlichen biologischen Fragmente mit mindestens 5 Aminosäuren, das in der Lage ist, die Polymerisation von Tubulin zu verändern.

2. Peptid nach Anspruch 1, mit einer Sequenz, die ausgewählt ist aus SEQ ID Nr. 5, SEQ ID Nr. 6, SEQ ID Nr. 7, SEQ ID Nr. 8, SEQ ID Nr. 17, SEQ ID Nr. 18, SEQ ID Nr. 19, SEQ ID Nr. 20, SEQ ID Nr. 21, SEQ ID Nr. 22, SEQ ID Nr. 23, SEQ ID Nr. 24, SEQ ID Nr. 25, SEQ ID Nr. 26, SEQ ID Nr. 27 oder SEQ ID Nr. 28.

3. Peptid nach einem der Ansprüche 1 und 2, **dadurch gekennzeichnet, dass** es durch Rekombination oder durch chemische Synthese erhalten ist.

4. Isolierte Nukleinsäure, die für ein Peptid nach einem der Ansprüche 1 bis 3 kodiert.

5. Nukleinsäure nach Anspruch 4, die nach optimalem Sequenzalignment mindestens 80 %, vorzugsweise 85 %, 90 %, 95 %, 98 % und 100 % Identität mit einer Sequenz aufweist, die ausgewählt ist aus SEQ ID Nr. 1, SEQ ID Nr. 2, SEQ ID Nr. 3, SEQ ID Nr. 4, SEQ ID Nr. 29, SEQ ID Nr. 30, SEQ ID Nr. 31, SEQ ID Nr. 32, SEQ ID Nr. 33, SEQ ID Nr. 34, SEQ ID Nr. 35, SEQ ID Nr. 36, SEQ ID Nr. 37, SEQ ID Nr. 38, SEQ ID Nr. 39 oder SEQ ID Nr. 40.

6. Vektor, **dadurch gekennzeichnet, dass** er eine Nukleinsäure nach einem der Ansprüche 4 und 5 enthält.

7. Wirtszelle, **dadurch gekennzeichnet, dass** sie mit einer Nukleinsäure nach einem der Ansprüche 4 und 5 oder einem Vektor nach Anspruch 6 transformiert ist.

8. Verfahren zur rekombinanten Herstellung des Peptids nach einem der Ansprüche 1 bis 3, **dadurch gekennzeichnet, dass** es die folgenden Schritte umfasst:
a) Kultivierung einer Zelle nach Anspruch 7 in einem Kulturmedium und unter geeigneten Kulturbedingungen,
b) Gewinnung des Peptids aus den Zellen oder aus dem in Schritt a) erhaltenen Kulturmedium.

9. Pharmazeutische Zusammensetzung, umfassend ein Peptid nach einem der Ansprüche 1 bis 3 und/oder erhalten durch ein Verfahren nach Anspruch 8, mit einem pharmazeutisch akzeptablen Träger.

10. Peptid nach einem der Ansprüche 1 bis 3 und/oder erhalten durch ein Verfahren nach Anspruch 8 für eine Verwendung zur Verhinderung des Zellwachstums und/oder der Zellbeweglichkeit zur Vorbeugung oder Behandlung einer Erkrankung, die ausgewählt ist aus Krebserkrankungen, bakteriellen Infektionen, Erkrankungen, die ein Wachstum bestimmter Pflanzen wie Algen oder Flechten mit sich bringen, oder Psoriasis.

11. Peptid nach Anspruch 10 für eine Verwendung zur Verhinderung der Angiogenese bei Tumorwachstum.

12. Peptid nach Anspruch 10 für eine Verwendung zur Verhinderung der bakteriellen Vermehrung.

13. Peptid nach einem der Ansprüche 1 bis 3 und/oder erhalten durch ein Verfahren nach Anspruch 8 für eine *in-vitro*-Verwendung als Tubulinmarker.

14. Peptid nach einem der Ansprüche 1 bis 3 und/oder erhalten durch ein Verfahren nach Anspruch 8 für eine Verwendung als Medikament.

15. Peptid nach Anspruch 14, **dadurch gekennzeichnet, dass** das Medikament zur Vorbeugung oder Behandlung von Erkrankungen vorgesehen ist, die ein Zellwachstum und/oder eine Zellmotilität mit sich bringen, nämlich bakterielle Infektionen, Erkrankungen, die ein Wachstum bestimmter Pflanzen wie Algen oder Flechten mit sich bringen, oder Psoriasis.

16. Peptid nach Anspruch 14, **dadurch gekennzeichnet, dass** das Medikament für die Vorbeugung oder Behandlung von Krebserkrankungen vorgesehen ist.

17. Peptid nach Anspruch 16, **dadurch gekennzeichnet, dass** die Krebserkrankung eine Krebserkrankung des Nervensystems ist.

18. Verwendung eines Peptids nach einem der Ansprüche 1 bis 3 und/oder erhalten durch ein Verfahren nach Anspruch 8 zur Erkennung und/oder Prüfung von Erzeugnissen *in vitro,* die in der Lage sind, die Interaktion zwischen Tubulin und dem Peptid zu blockieren.

19. Verwendung eines Fragments eines Intermediärfilaments, das in der Lage ist, die Polymerisation von Tubulin zu verändern, ausgewählt aus Endo-A-Cytokeratin, das nach optimalem Sequenzalignment mindestens 80 %, vorzugsweise 85 %, 90 %, 98 % und 100 % Identität mit der Sequenz SEQ ID Nr. 9 aufweist, Keratin 8, das nach optimalem Sequenzalignment mindestens 80 %, vorzugsweise 85 %, 90 %, 98 % und 100 % Identität mit der Sequenz SEQ ID Nr. 10 aufweist, Peripherin, das nach optimalem Sequenzalignment mindestens 80 %, vorzugsweise 85 %, 90 %, 98 % und 100 % Identität mit der Sequenz SEQ ID Nr. 11 aufweist, Internexin, das nach optimalem Sequenzalignment mindestens 80 %, vorzugsweise 85 %, 90 %, 98 % und 100 % Identität mit der Sequenz SEQ ID Nr. 12 aufweist, GFAP, das nach optimalem Sequenzalignment mindestens 80 %, vorzugsweise 85 %, 90 %, 98 % und 100 % Identität mit der Sequenz SEQ ID Nr. 13 aufweist, oder Vimentin, das nach optimalem Sequenzalignment mindestens 80 %, vorzugsweise 85 %, 90 %, 98 % und 100 % Identität mit der Sequenz SEQ ID Nr. 14 aufweist, für die Herstellung eines Medikaments, das zur Vorbeugung oder Behandlung von Erkrankungen vorgesehen ist, die ein Zellwachstum und/oder eine Zellbeweglichkeit mit sich bringen, ausgewählt aus Krebserkrankungen, bakteriellen Infektionen, Erkrankungen, die ein Wachstum bestimmter Pflanzen wie Algen oder Flechten mit sich bringen, oder Psoriasis.

20. *In-vitro*-Verwendung eines Fragmente eines Intermediärfilaments, das in der Lage ist, die Polymerisation von Tubulin zu verändern, ausgewählt aus Endo-A-Cytokeratin, das nach optimalem Sequenzalignment mindestens 80 %, vorzugsweise 85 %, 90 %, 98 % und 100 % Identität mit der Sequenz SEQ ID Nr. 9 aufweist, Keratin 8, das nach optimalem Sequenzalignment mindestens 80 %, vorzugsweise 85 %, 90 %, 98 % und 100 % Identität mit der Sequenz SEQ ID Nr. 10 aufweist, Peripherin, das nach optimalem Sequenzalignment mindestens 80 %, vorzugsweise 85 %, 90 %, 98 % und 100 % Identität mit der Sequenz SEQ ID Nr. 11 aufweist, Internexin, das nach optimalem Sequenzalignment mindestens 80 %, vorzugsweise 85 %, 90 %, 98 % und 100 % Identität mit der Sequenz SEQ ID Nr. 12 aufweist, GFAP, das nach optimalem Sequenzalignment mindestens 80 %, vorzugsweise 85 %, 90 %, 98 % und 100 % Identität mit der Sequenz SEQ ID Nr. 13 aufweist, oder Vimentin, das nach optimalem Sequenzalignment mindestens 80 %, vorzugsweise 85 %, 90 %, 98 % und 100 % Identität mit der Sequenz SEQ ID Nr. 14 aufweist, als Tubulinmarker.

21. Fragment eines Intermediärfilaments, das in der Lage ist, die Polymerisation von Tubulin zu verändern, ausgewählt aus Endo-A-Cytokeratin, das nach optimalem Sequenzalignment mindestens 80 %, vorzugsweise 85 %, 90 %, 98 % und 100 % Identität mit der Sequenz SEQ ID Nr. 9 aufweist, Keratin 8, das nach optimalem Sequenzalignment mindestens 80 %, vorzugsweise 85 %, 90 %, 98 % und 100 % Identität mit der Sequenz SEQ ID Nr. 10 aufweist, Peripherin, das nach optimalem Sequenzalignment mindestens 80 %, vorzugsweise 85 %, 90 %, 98 % und 100 % Identität mit der Sequenz SEQ ID Nr. 11 aufweist, Internexin, das nach optimalem Sequenzalignment mindestens 80 %, vorzugsweise 85 %, 90 %, 98 % und 100 % Identität mit der Sequenz SEQ ID Nr. 12 aufweist, GFAP, das nach optimalem Sequenzalignment mindestens 80 %, vorzugsweise 85 %, 90 %, 98 % und 100 % Identität mit der Sequenz SEQ ID Nr. 13 aufweist, oder Vimentin, das nach optimalem Sequenzalignment mindestens 80 %, vorzugsweise 85 %, 90 %, 98 % und 100 % Identität mit der Sequenz SEQ ID Nr. 14 aufweist, für eine Verwendung als Medikament.

22. Verwendung eines Fragments eines Intermediärfilaments, das in der Lage ist, die Polymerisation von Tubulin zu verändern, ausgewählt aus Endo-A-Cytokeratin, das nach optimalem Sequenzalignment mindestens 80 %, vorzugsweise 85 %, 90 %, 98 % und 100 % Identität mit der Sequenz SEQ ID Nr. 9 aufweist, Keratin 8, das nach optimalem Sequenzalignment mindestens 80 %, vorzugsweise 85 %, 90 %, 98 % und 100 % Identität mit der Sequenz SEQ ID Nr. 10 aufweist, Peripherin, das nach optimalem Sequenzalignment mindestens 80 %, vorzugsweise 85 %, 90 %, 98 % und 100 % Identität mit der Sequenz SEQ ID Nr. 11 aufweist, Internexin, das nach optimalem Sequenzalignment mindestens 80 %, vorzugsweise 85 %, 90 %, 98 % und 100 % Identität mit der Sequenz SEQ ID Nr. 12 aufweist, GFAP, das nach optimalem Sequenzalignment mindestens 80 %, vorzugsweise 85 %, 90 %, 98 % und 100 % Identität mit der Sequenz SEQ ID Nr. 13 aufweist, oder Vimentin, das nach optimalem Sequenzalignment mindestens 80 %, vorzugsweise 85 %, 90 %, 98 % und 100 % Identität mit der Sequenz SEQ ID Nr. 14 aufweist, zur Erkennung und/oder Prüfung von Erzeugnissen *in vitro,* die geeignet sind, die Interaktion zwischen Tubulin und dem Fragment eines Intermediärfilaments zu blockieren.


### LISTE DE SEQUENCES

<110> INSERM (Institut National de la Santé Et de la Recherche Médicale)
   Université d'Angers
   McGill University
<120> Peptide capable d'altérer la polymérisation de la tubuline et son utilisation pour inhiber la prolifération cellulaire
<130> D22155
<150> FR 04/04 742
   <151> 2004-05-04
<160> 40
<170> PatentIn version 3.2
<210> 1
   <211> 72
   <212> ADN
   <213> mus musculus
<400> 1
   ttcggctacg atccgtactt ttcgacctcc tacaagcggc gctatgtgga gacgccccgg     60
   gtgcacatct cc                                                         72
<210> 2
   <211> 72
   <212> ADN
   <213> mus musculus
<400> 2
   actccgcgcc ggtctcctcc tcgctgtccg tgcgccgcag ctactcgtcc agctctggct     60
   ctttgatgcc ca                                                         72
<210> 3
   <211> 117
   <212> ADN
   <213> mus musculus
<400> 3
   gcctaccggc gcgttccaac cgagacccgg tccagcttca gccgcgtgag cggttccccg     60
   tccagcggct tccgctcgca gtcctggtcc cgcggctcgc ccagcaccgt gtcctcc      117
<210> 4
   <211> 117
   <212> ADN
   <213> mus musculus
<400> 4
   cgctccgcgg ccggctcctc cagcggcttc cactcgtggg cgcggacgtc cgtgagctcc      60
   gtgtccgcct cacccagccg cttccgcggc gccgcctcga gcaccgactc gctagac        117
<210> 5
<211> 24
   <212> PRT
   <213> mus musculus
<400> 5
   Phe Gly Tyr Asp Pro Tyr Phe Ser Thr Ser Tyr Lys Arg Arg Tyr Val
   1               5                   10                  15
   Glu Thr Pro Arg Val His Ile Ser
               20
<210> 6
   <211> 24
   <212> PRT
   <213> mus musculus
<400> 6
   Tyr Ser Ser Tyr Ser Ala Pro Val Ser Ser Ser Leu Ser Val Arg Arg
   1               5                   10                  15
   Ser Tyr Ser Ser Ser Ser Gly Ser
               20
<210> 7
   <211> 39
   <212> PRT
   <213> mus musculus
<400> 7
   Ala Tyr Arg Arg Val Pro Thr Glu Thr Arg Ser Ser Phe Ser Arg Val
   1               5                   10                  15
   Ser Gly Ser Pro Ser Ser Gly Phe Arg Ser Gln Ser Trp Ser Arg Gly
               20                  25                  30
   Ser Pro Ser Thr Val Ser Ser
           35
<210> 8
   <211> 39
   <212> PRT
   <213> mus musculus
<400> 8
   Arg Ser Ala Ala Gly Ser Ser Ser Gly Phe His Ser Trp Ala Arg Thr
   1               5                   10                  15
   Ser Val Ser Ser Val Ser Ala Ser Pro Ser Arg Phe Arg Gly Ala Ala
               20                  25                  30
   Ser Ser Thr Asp Ser Leu Asp
           35
<210> 9
   <211> 38
   <212> PRT
   <213> mus musculus
<400> 9
   Lys Ser Tyr Lys Met Ser Thr Ser Cys Pro Arg Ala Phe Ser Ser Arg
   1               5                   10                  15
   Ser Phe Thr Ser Cys Pro Cys Ala Arg Ile Ser Ser Ser Ser Pro Ser
               20                  25                  30
   Arg Val Cys Ser Ser Ser
           35
<210> 10
   <211> 38
   <212> PRT
   <213> mus musculus
<400> 10
   Lys Ser Tyr Lys Met Ser Thr Ser Cys Pro Arg Ala Phe Ser Ser Arg
   1               5                   10                  15
   Ser Phe Thr Ser Cys Pro Cys Ala Arg Ile Ser Ser Ser Ser Pro Ser
               20                  25                  30
   Arg Val Cys Ser Ser Ser
           35
<210> 11
   <211> 38
   <212> PRT
   <213> mus musculus
<400> 11
   Arg Phe Ser Ser Ser Arg Leu Leu Gly Ser Gly Ser Pro Ser Ser Ser
   1               5                   10                  15
   Ala Arg Leu Gly Ser Phe Arg Ala Pro Arg Ala Gly Ala Leu Arg Leu
               20                  25                  30
   Pro Ser Glu Arg Leu Asp
           35
<210> 12
   <211> 27
   <212> PRT
   <213> mus musculus
<400> 12
   Met Ser Phe Gly Ser Glu His Tyr Leu Cys Ser Ala Ser Ser Tyr Arg
     1               5                   10                  15
   Lys Val Phe Gly Asp Ser Ser Arg Leu Ser Ala
               20                  25
<210> 13
   <211> 23
   <212> PRT
   <213> mus musculus
<400> 13
   Arg Gln Leu Gly Thr Met Pro Arg Phe Ser Leu Ser Arg Met Thr Pro
   1               5                   10                  15
   Pro Leu Pro Ala Arg Val Asp
               20
<210> 14
   <211> 41
   <212> PRT
   <213> mus musculus
<400> 14
   Arg Pro Ser Thr Ser Arg Ser Leu Tyr Ser Ser Ser Pro Gly Gly Ala
   1               5                   10                  15
   Tyr Val Thr Arg Ser Ser Ala Val Arg Leu Arg Ser Ser Val Pro Gly
               20                  25                  30
   Val Arg Leu Leu Gln Asp Ser Val Asp
           35                  40
<210> 15
   <211> 20
   <212> ADN
   <213> Séquence artificielle
<220>
   <223> Amorce
<400> 15
   ctgcaaggcg attaagttgg 20
<210> 16
   <211> 20
   <212> ADN
   <213> Séquence artificielle
<220>
   <223> Amorce
<400> 16
   gttgtgtgga attgtgagcg 20
<210> 17
   <211> 27
   <212> PRT
   <213> Mus musculus
<400> 17
   Met Ser Gln Ala Tyr Ser Ser Ser Gln Arg Val Ser Ser Tyr Arg Arg
   1               5                   10                  15
   Thr Phe Gly Gly Ala Pro Gly Phe Ser Leu Gly
               20                  25
<210> 18
   <211> 54
   <212> PRT
   <213> Mus musculus
<400> 18
   Ser Ser Pro Val Phe Pro Arg Ala Gly Phe Gly Thr Lys Gly Ser Ser
   1               5                   10                  15
   Ser Ser Met Thr Ser Arg Val Tyr Gln Val Ser Arg Thr Ser Gly Gly
               20                  25                  30
   Ala Gly Gly Leu Gly Ser Leu Arg Ser Ser Arg Leu Gly Thr Thr Arg
           35                  40                  45
   Ala Pro Ser Tyr Gly Ala
       50
<210> 19
   <211> 21
   <212> PRT
   <213> Mus musculus
<400> 19
   Gly Ser Glu Val His Thr Lys Lys Thr Val Met Ile Lys Thr Ile Glu
   1               5                   10                  15
   Thr Arg Asp Gly Glu
               20
<210> 20
   <211> 18
   <212> PRT
   <213> Mus musculus
<400> 20
   Met Ser Thr Arg Ser Val Ser Ser Ser Ser Tyr Arg Arg Met Phe Gly
   1               5                   10                  15
   Gly Ser
<210> 21
   <211> 24
   <212> PRT
   <213> Mus musculus
<400> 21
   Gly Gly Ala Tyr Val Thr Arg Ser Ser Ala Val Arg Leu Arg Ser Ser
   1               5                   10                  15
   Val Pro Gly Val Arg Leu Leu Gln
               20
<210> 22
   <211> 21
   <212> PRT
   <213> Mus musculus
<400> 22
   Ser Leu Pro Leu Val Asp Thr His Ser Lys Arg Thr Leu Leu Ile Lys
   1               5                   10                  15
   Thr Val Glu Thr Arg
               20
<210> 23
   <211> 54
   <212> PRT
   <213> Mus musculus
<400> 23
   Met Ser Ile Arg Val Thr Gln Lys Ser Tyr Lys Met Ser Thr Ser Gly
   1 5 10 15
   Pro Arg Ala Phe Ser Ser Arg Ser Phe Thr Ser Gly Pro Gly Ala Arg
   20 25 30
   Ile Ser Ser Ser Ser Phe Ser Arg Val Gly Ser Ser Ser Ser Ser Phe
   35 40 45
   Arg Gly Ser Met Gly Thr
   50
<210> 24
   <211> 21
   <212> PRT
   <213> Mus musculus
<400> 24
   Gln Ile Lys Ser Leu Asn Asn Lys Phe Ala Ser Phe Ile Asp Lys Val
   1 5 10 15
   Arg Phe Leu Glu Gln
   20
<210> 25
   <211> 30
   <212> PRT
   <213> Mus musculus
<400> 25
   Ser Ala Gly Gly Ser Asn Thr Phe Ser Arg Thr Thr Lys Ala Val Val
   1 5 10 15
   Val Lys Lys Ile Glu Thr Arg Asp Gly Lys Leu Val Ser Glu
   20 25 30
<210> 26
   <211> 24
   <212> PRT
   <213> Mus musculus
<400> 26
   Met Glu Arg Arg Arg Ile Thr Ser Ala Arg Arg Ser Tyr Ala Ser Glu
   1 5 10 15
   Thr Val Val Arg Gly Leu Gly Pro
   20
<210> 27
   <211> 36
   <212> PRT
   <213> Mus musculus
<400> 27
   Val Arg Gly Leu Gly Pro Ser Arg Gln Leu Gly Thr Met Pro Arg Phe
   1               5                   10                  15
   Ser Leu Ser Arg Met Thr Pro Pro Leu Pro Ala Arg Val Asp Phe Ser
               20                  25                  30
   Leu Ala Gly Ala
           35
<210> 28
   <211> 21
   <212> PRT
   <213> Mus musculus
<400> 28
   Lys Ser Val Ser Glu Gly His Leu Lys Arg Asn Ile Val Val Lys Thr
   1               5                   10                  15
   Val Glu Met Arg Asp
               20
<210> 29
   <211> 81
   <212> ADN
   <213> Mus musculus
<400> 29
   atgagccagg cctactcgtc cagccagcgc gtgtcctcct accgccgcac cttcggcggc      60
   gccccgggct tctctctggg c                                                81
<210> 30
   <211> 166
   <212> ADN
   <213> Mus musculus
<400> 30
   gagctctccc gtgttccctc gagcaggctt cggtaccaag ggctcctcga gttcaatgac      60
   atcccgcgtg taccaggtgt cgcgcacgtc gggcggggct ggaggcttgg ggtcgctgcg     120
   gtctagccgg ctggggacca cccgagcgcc atcctatggc gcgggc                    166
<210> 31
   <211> 61
   <212> ADN
   <213> Mus musculus
<400> 31
   ttctgaagtc cataccaaaa agacagtgat gatcaagacc attgagaccc gggatggaga 60
   g 61
<210> 32
   <211> 54
   <212> ADN
   <213> Mus musculus
<400> 32
   atgtctacca ggtctgtgtc ctcgtcctcc taccgcagga tgttcggtgg ctcc 54
<210> 33
   <211> 73
   <212> ADN
   <213> Mus musculus
<400> 33
   cggtggcgcc tatgtgaccc ggtcctcggc agtgcgcctg cggagcagcg tgccgggcgt 60
   gcggctgctt caa 73
<210> 34
   <211> 60
   <212> ADN
   <213> Mus musculus
<400> 34
   cttcctctgg ttgacaccca ctcaaaaaga acactcctga ttaagacggt tgagaccaga 60
<210> 35
   <211> 161
   <212> ADN
   <213> Mus musculus
<400> 35
   tgtccatcag ggtgactcag aaatcctaca agatgtccac ctccggtccc cgggccttca 60
   gcagccgctc gttcacgagt ggacccggtg cccgcatcag ctcttccagc ttttcccggg 120
   tgggcagcag cagcagcagc ttccggggaa gcatgggcac c 161
<210> 36
   <211> 61
   <212> ADN
   <213> Mus musculus
<400> 36
   gattaaatcc ctgaacaaca agttcgcctc cttcattgac aaggtgcgct tcctggagca 60
   g 61
<210> 37
   <211> 88
   <212> ADN
   <213> Mus musculus
<400> 37
   tgccggggga tccaacactt tcagccgcac caccaaggct gtggttgtga agaagattga     60
   aacccgagat gggaagctgg tgtccgag                                        88
<210> 38
   <211> 72
   <212> ADN
   <213> Mus musculus
<400> 38
   atgcctccga gacggtggtc aggggcctct ggtcctagtc gacaactggg taccatgcca     60
   cgtttctcct tg                                                         72
<210> 39
   <211> 106
   <212> ADN
   <213> Mus musculus
<400> 39
   gccacgtttc tccttgtctc gaatgactcc tccactccct gccagggtgg acttctccct     60
   ggccggggcg ctcaatgctg gcttcaagga gacacgggcg agcgag                   106
<210> 40
   <211> 61
   <212> ADN
   <213> Mus musculus
<400> 40
   ggtaaagact gtggagatgc gggatggtga ggtcattaag gactcgaagc aggagcacaa     60
   g                                                                     61